# EUROPÄISCHE PATENTSCHRIFT

(11) **EP 0 885 614 B1**
(45) Veröffentlichungstag und Bekanntmachung des Hinweises auf die Patenterteilung: **27.09.2000**
(21) Anmeldenummer: 98110972.1
(22) Anmeldetag: 16.06.1998
(51) Int. Cl.: A61K 39/395, A61K 45/00

(54) **Verfahren zur ex vivo-Immunisierung mittels heterologer intakter bispezifischer und/oder trispezifischer Antikörper**
Method of ex vivo immunizing using heterologous intact bispecific and/or trispecific antibodies
Procédé d'immunisation ex vivo utilisant des anticorps hétérologues intactes bispécifiques ou trispécifiques

(30) Priorität: 17.06.1997 DE 19725586
(43) Veröffentlichungstag der Anmeldung: 23.12.1998
(73) Patentinhaber: GSF-Forschungszentrum für Umwelt und Gesundheit, GmbH, 85764 Oberschleissheim (DE)
(72) Erfinder: Lindhofer, Horst, Dr., 82194 Gröbenzell (DE); Kolb, Hans-Jochem, Prof. Dr., 80804 München (DE); Zeidler, Reinhard, Dr., 81249 München (DE); Bornkamm, Georg, Prof. Dr., 81243 München (DE)
(74) Vertreter: Reinhard - Skuhra - Weise & Partner

(56) Entgegenhaltungen:
- EP-A- 0 826 695
- LINDHOFER, HORST ET AL: "Bispecific antibodies target operationally tumor-specific antigens in two leukemia relapse models" BLOOD (1996), 88(12), 4651-4658 CODEN: BLOOAW;ISSN: 0006-4971, XP000616201
- LINDHOFER H ET AL: "Increased tumor-specificity and -elimination by selectively binding bispecific antibodies in vivo." 25TH ANNUAL MEETING OF THE INTERNATIONAL SOCIETY FOR EXPERIMENTAL HEMATOLOGY, NEW YORK, NEW YORK, USA, AUGUST 23-27, 1996. EXPERIMENTAL HEMATOLOGY (CHARLOTTESVILLE) 24 (9). 1996. 1090. ABSTRACT 348. ISSN: 0301-472X, XP002084410
- WOLLENBERG B ET AL: "A bispecific antibody induces efficient killing of tumor cells: Phase I-trial in patients with HNSCC." INTERNATIONAL SYMPOSIUM ON METASTASES IN HEAD AND NECK CANCER, KIEL, GERMANY, JANUARY 15-18, 1998. BRITISH JOURNAL OF CANCER 77 (SUPPL. 1). 1998. 46. ABSTRACT 15.16. ISSN: 0007-0920, XP002084411
- LINDHOFER H ET AL: "Bispecific antibodies effectively purge cancer cells from peripheral blood stem cell collections without affecting colony forming units." 26TH ANNUAL MEETING OF THE INTERNATIONAL SOCIETY FOR EXPERIMENTAL HEMATOLOGY, CANNES, FRANCE, AUGUST 24-28, 1997. EXPERIMENTAL HEMATOLOGY (CHARLOTTESVILLE) 25 (8). 1997. 879. ABSTRACT 527. ISSN: 0301-472X, XP002048523

## Beschreibung

Die Erfindung betrifft ein Verfahren zur Herstellung eines Tumorzellpräparats zur Immunisierung mittels heterologer, intakter bispezifischer und/oder trispezifischer Antikörper sowie Verwendungen der Produkte dieses Verfahrens zur Prophylaxe und Therapie von Tumorerkrankungen, insbesondere zur Induktion einer Tumorimmunität.

Maligne Erkrankungen des Menschen, beispielsweise Brustkrebs im fortgeschrittenen Stadium, haben trotz der Fortschritte der Chemo- und Radiotherapie in den letzten Jahren noch immer eine äußerst ungünstige Prognose. Eine Heilung dieser Erkrankungen ist in der Regel nicht möglich. Es ist daher notwendig, neue Behandlungsstrategien zu entwickeln. Große Hoffnungen werden dabei auf immuntherapeutische Ansätze gesetzt, mittles derer das Immunsystem des Patienten dazu gebracht werden soll, den Tumor abzustoßen. Es ist bekannt, daß auf Tumorzellen tumorassoziierte Antigene vorkommen und daß das Immunsystem prinzipiell durchaus in der Lage ist, diese zu erkennen und die malignen Zellen anzugreifen. Tumoren haben jedoch verschiedene Strategien entwickelt, die es ihnen erlauben, sich der Immunantwort zu entziehen. Dies gelingt ihnen z.B. durch ungenügende Präsentation von tumorassoziierten Antigenen und/oder durch unzureichende Aktivierung der in der Regel vorhandenen tumorspezifischen T-Zellen.

Bei etwa 43.000 Neuerkrankungen / Jahr steht Brustkrebs an der Spitze der Krebsstatistik für Frauen in Deutschland. Weniger als ein Drittel der Frauen mit Lymphknotenbefall bei Diagnose leben 10 Jahre ohne Rückfall.

Immuntherapeutische Ansätze beim Mamma-Karzinom beschränkten sich bisher auf Methoden zur unspezifischen Stimulation wie die Behandlung mit BCG oder Levamisol, sowie den Einsatz von LAK- und NK-Zellen mit IL-2 (3, 4). Eine Lebensverlängerung konnte mit bisher eingesetzten Formen von Immuntherapie nicht nachgewiesen werden, die Behandlung mit BCG war sogar eher nachteilig (3). Nachdem unspezifische Aktivierungen von Zellen auch bei anderen Tumorarten wenig Erfolg gehabt haben, sollte nun versucht werden, eine spezifische Immunreaktion in Gang zu bringen.

Für die Tumortherapie wurden beispielsweise T-Zell-redirigierende bispezifische Antikörper verwendet. Diese binden mit einem Bindungsarm an einen T-Zell-Rezeptorkomplex und mit dem zweiten Bindungsarm an ein tumorassoziiertes Antigen auf einer Tumorzelle. Durch die daraus resultierende Aktivierung der T-Zelle und der räumlichen Nähe der Tumorzelle wird letztere durch Apoptose-Induktion bzw. Zytokine wie TNF-α oder Perforin zerstört.

Aus Blood, 88(12), 1996, 4651-8 ist bekannt, daß bispezifische Antikörper operationell gegen tumorspezifische Antigene bei der Leukämiebehandlung gerichtet werden können. Hierbei wurden bestrahlte BALB/c (Thy-1.2⁺, I-A^{d})-Mäuse mit C57BL/6 Thy-1.1 (I-A^{b}) BM-Zellen unter solchen Bedingungen transplantiert, daß eine Graft-versus-Host-Erkrankung durch monoclonale Antikörper verhindert wurde. Diesen chimeren Mäusen wurden entweder 2 x 10⁴ Thy-1.2⁺, CD3⁻ ST-1-Zellen vom Empfängertyp oder MHC-Klasse II⁺ (I-A^{d})-BCL1-Zellen injiziert. Nach vier Tagen wurden die Mäuse mit dem bispezifischen Antikörper G2 (anti-CD3 x anti-Thy-1.2) oder dem bispezifischen Antikörper BiC (anti-CD3 x anti-l-A^{d}) behandelt. Hierdurch wurden ausschließlich die Targetarme der bispezifischen Antikörper an die Tumorzellen gebunden und die hematopoetischen Zellen vom Spendertyp wurden nicht gebunden. Im Vergleich mit den parentalen Antikörpern verringerten die bispezifischen Antikörper die Mortalität durch Tumoren signifikant. Während in den Kontrollgruppen 0% der mit den parentalen Antikörpern behandelten Tiere überlebten, überlebten in den mit den bispezifischen Antikörper-Gruppen behandelten Mäusen 34% bzw. 83%.

Experimental Hematology, 24(9), 1996, 1090, Abstract 348 beschreibt, daß bispezifische Antikörper in vivo selektiv an Tumoren binden und Tumoren eliminieren können. Eine ex vivo-Herstellung eines Tumorzellpräparats mit den Schritten des Anspruchs 1 wird nicht beschrieben.

Die im Stand der Technik zur Tumortherapie verwendeten bispezifischen Antikörper wurden direkt in die Patienten infundiert. Diese Vorgehensweise weist mehrere Nachteile auf:
- Es sind hohe Antikörper-Dosen notwendig;
- es können gravierende Nebenwirkungen auftreten;
- durch den Tumorbindungsarm können die Antikörper bei in vivo-Applikation auch an Normalgewebe binden.

Es ist eine Aufgabe der vorliegenden Erfindung, ein neues Verfahren zur Herstellung eines Tumorzellpräparats bereitzustellen, um maligne Erkrankungen des Menschen, insbesondere mit dem Ziel einer Tumorimmunität, zu therapieren.

Diese Aufgabe wird erfindungsgemäß durch das im Anspruch 1 näher gekennzeichnete Verfahren gelöst. Bevorzugte Ausgestaltungen des Verfahrens ergeben sich auch aus den Unteransprüchen.

Weitere Ansprüche betreffen die Verwendung des erfindungsgemäß bereitgestellten Tumorzellpräparats zur Prophylaxe und Therapie von Tumorerkrankungen, insbesondere zur Erreichung einer Tumor immunität, besonders bevorzugt einer Langzeit-Immunität.

Das Endprodukt des erfindungsgemäßen Verfahrens ist eine pharmazeutische Zubereitung, die Tumorzellen und Antikörper enthält. Diese Tumorzellzubereitung wird erfindungsgemäß zur Prophylaxe und Behandlung von Tumorerkrankungen eingesetzt, um eine Tumorimmunität, bevorzugt eine Langzeit-Tumorimmunität, zu erreichen.

Erfindungsgemäß werden autologe Tumorzellen mit heterologen bispezifischen und/oder trispezifischen Antikörpern behandelt, und die so erhaltene Tumorzellzusammensetzung wird in den Patienten oder in Tiere, aus denen die autologen Tumorzellen isoliert wurden, reinfundiert.

Die erfindungsgemäß durchgeführten Versuche, insbesondere das hier beschriebene Beispiel 2, zeigen, daß eine langanhaltende Tumorimmunität erreichbar ist. Die in Mäusen durchgeführten Untersuchungen und die derart erhaltenen Ergebnisse können auch auf Menschen übertragen werden. Es ist davon auszugehen, daß eine mehrjährige und damit eine langandauernde Tumorimmunität durch die Anwendung der durch das erfindungsgemäße Verfahren hergestellten Tumorzellpräparate erreichbar ist.

Erfindungsgemäß versteht man unter einer Tumorzelle jede Zelle, die durch eine oder mehrere Mutationen ihre normale Funktion verloren oder verändert hat und sich unkontrolliert vermehren kann.

Die erfindungsgemäß erreichbare Tumorimmunität ist dadurch gekennzeichnet, daß in einem Organismus das körpereigene Immunsystem gegen den autologen Tumor derart aktiviert wird, daß es zu einer dauerhaften Zerstörung und/oder Kontrolle des autologen Tumors kommt.

Erfindungsgemäß ist jede Art von Tumoren behandelbar, die unter die oben angegebene Definition fällt. Insbesondere therapierbar sind epitheliale Tumore, Adenokarzinome, Kolonkarzinome, Mammakarzinome, Ovarialkarzinome, Lungenkarzinome und Hals-, Nasen- und/oder Ohrentumore. Weiterhin behandelbar sind nicht-epitheliala Tumoren wie Leukämien und Lymphome. Weiterhin therapierbar sind virusinduzierte Tumoren, beispielsweise Lebertumore oder Cervixkarzinome.

Mit dem erfindungsgemäßen Verfahren werden Tumorzellpräparate erhalten, die in Form pharmazeutischer Zubereitungen wieder an den Patienten verabreicht werden.

Erfindungsgemäß werden heterologe intakte bispezifische und/oder trispezifische Antikörper verwendet. Diese werden ex vivo mit zuvor aus einem Patienten entnommenen Tumorzellen (autologe Tumorzellen) in Kontakt gebracht. Um ein Überleben der Tumorzellen nach Reinfusion zu verhindern, wurden die Tumorzellen vor dem Inkontaktbringen mit den Antikörpern in an sich bekannter Weise behandelt, beispielsweise durch Bestrahlung. Die Tumorzellen werden nach Bestrahlung mit den intakten heterologen bispezifischen und/oder trispezifischen Antikörpern inkubiert. Erfindungsgemäß sind nicht beliebige Antikörper verwendbar, sondern diese müssen intakt sein, d.h. sie müssen einen funktionellen Fc-Teil besitzen, und sie müssen heterologer Natur sein, d.h. die Antikörper sind dann aus schweren Immunglobulinketten unterschiedlicher Subklassen (-Kombinationen, auch Fragmente) und/oder Herkunft (Spezies) zusammengesetzt.

Diese intakten heterologen bispezifischen und/oder trispezifischen Antikörper werden so ausgewählt, daß sie weiterhin die nachfolgenden Eigenschaften aufweisen:
- α -: Binden an eine T-Zelle;
- β -: Binden an zumindest ein Antigen auf einer Tumorzelle;
- γ -: Binden durch ihren Fc-Teil (bei bispezifischen Antikörpern) oder durch eine dritte Spezifität (bei trispezifischen Antikörpern) an Fc-Rezeptor positive Zellen.

In einer bevorzugten Ausführungsform der vorliegenden Erfindung werden bispezifische und/oder trispezifische Antikörper eingesetzt, die in der Lage sind, die Fc-Rezeptor-positive Zelle zu aktivieren. Hierdurch wird die Expression von Cytokinen und/oder co-stimulatorischen Antigenen iniziiert oder erhöht.

Bei den trispezifischen Antikörpern erfolgt die Bindung an die Fc-Rezeptor positiven Zellen bevorzugt beispielsweise über den Fc-Rezeptor von Fc-Rezeptor positiven Zellen oder auch über andere Antigene auf Fc-Rezeptor positiven Zellen (Antigen-präsentierenden Zellen), wie z.B. den Mannose-Rezeptor.

Nur durch das erfindungsgemäße Verfahren und den Einsatz der hier beschriebenen Antikörper ist gewährleistet, daß nach Verabreichung des nach dem erfindungsgemäßen Verfahren hergestellten Tumorzellpräparats an den Patienten, aus dem die Tumorzellen zuvor entnommen wurden, eine Tumorimmunität aufgebaut wird. Die Applikation (Reinfusion) erfolgt bevorzugt an einen Patienten nach der Behandlung des Primärtumors, bevorzugt bei Patienten in einer minimal residual disease (MRD)-Situation. Bei Patienten mit wenig verbliebenen Tumorzellen, bei denen allerdings die Gefahr eines Rezidivs hoch sein kann, wird das erfindungsgemäß bereitgestellte Verfahren besonders erfolgreich anwendbar sein.

Durch Anwendung der durch das erfindungsgemäße Verfahren hergestellten Tumorzellpräparate können die aus dem Stand der Technik bekannten und oben näher beschriebenen Nachteile vermieden werden.

Die erfindungsgemäß verwendbaren heterologen bispezifischen und/oder trispezifischen Antikörper sind zum Teil an sich bekannt, zum Teil werden sie aber auch in der vorstehenden Anmeldung zum ersten Mal beschrieben. Ein Beispiel für einen bsAk ist der Antikörper anti-CD3 X anti-epcam, der bei epithelialen Tumoren wie dem Mammacarcinom eingesetzt wird.

Erfindungsgemäß werden zwei Verfahrens-Varianten unterschieden:
1. Kurzzeitinkubation, und
2. Langzeitinkubation

Bei der Kurzzeitinkubation werden die autologen Tumorzellen mit den intakten heterologen bispezifischen und/oder trispezifischen Antikörpern für einen Zeitraum von 10 Minuten bis 5 Stunden oder 10 Minuten bis 3 Stunden oder weiterhin bevorzugt für einen Zeitraum von ca. 15 Minuten bis 2 Stunden, weiterhin bevorzugt für einen Zeitraum von 15 Minuten bis 1 Stunde, inkubiert. Die dann mit den Antikörpern beladenen Tumorzellen werden dann zur Reinfusion vorbereitet.

Bei der Langzeitinkubation werden die autologen Tumorzellen ebenfalls für einen Zeitraum von ca. 10 Minuten bis 5 Stunden, bevorzugt für einen Zeitraum von 15 Minuten bis 2 Stunden und weiterhin bevorzugt für einen Zeitraum von 15 Minuten bis 1 Stunde, inkubiert, so daß die autologen Tumorzellen mit den Antikörpern beladen werden. Anschließend werden hierzu Blutzellen des Patienten, bevorzugt mononukleäre Zellen aus dem peripheren Blut (PBMC = peripheral blood mononucleated cells), zugegeben, und diese Mischung wird dann über einen längeren Zeitraum, beispielsweise 1 bis 14 Tage lang, bevorzugt 3 bis 10 Tage und weiterhin bevorzugt 6 bis 10 Tage, inkubiert. Alternativ hierzu können in einer weiteren Verfahrensführung die autologen Tumorzellen unmittelbar mit den bispezifischen und/oder trispezifischen Antikörpern und mit den Blutzellen des Patienten, bevorzugt den mononukleären Zellen aus dem peripheren Blut, in Kontakt gebracht werden. Hierdurch wird eine Vielzahl von Immunzellen bereits ex vivo gegen den Tumor "geprimt". Anschließend werden diese in den Patienten reinfundiert. Durch die Langzeitinkubation wird auch eine Internalisierung der Antikörper und ihr Abbau erreicht.

Erste in vitro-Ergebnisse zeigen, daß durch derartig vorbehandelte Immunzellen Tumorzellen ohne weitere Zugabe von bispezifischen und/oder trispezifischen Antikörpern zerstört werden können (vgl. Beispiel 1).

Sowohl bei der Kurzzeitinkubation als auch bei der Langzeitinkubation werden T-Zellen an die Tumorzellen mittels der an den Tumorzellen immobilisierten intakten bispezifischen und/oder trispezifischen Antikörper redirigiert; gleichzeitig findet eine Bindung von Fc-Rezeptor positiven Zellen an den Fc-Teil des bispezifischen und/oder trispezifischen Antikörpers nach Reinfusion statt. Dabei werden die Fc-Rezeptor positiven Zellen durch Bindung an die Fc-Teile von (an der T-Zelle bzw. Tumorzelle)immobilisierten intakten bispezifischen Antikörpern aktiviert.

Zur Verbesserung des Immunisierungserfolges können die entweder nach dem Kurzzeit-Inkubationsverfahren oder nach dem Langzeit-Inkubationsverfahren mit den Antikörpern behandelten Tumorzellen nicht nur einmal an den Patienten appliziert, sondern wahlweise auch mehrfach verabreicht werden.

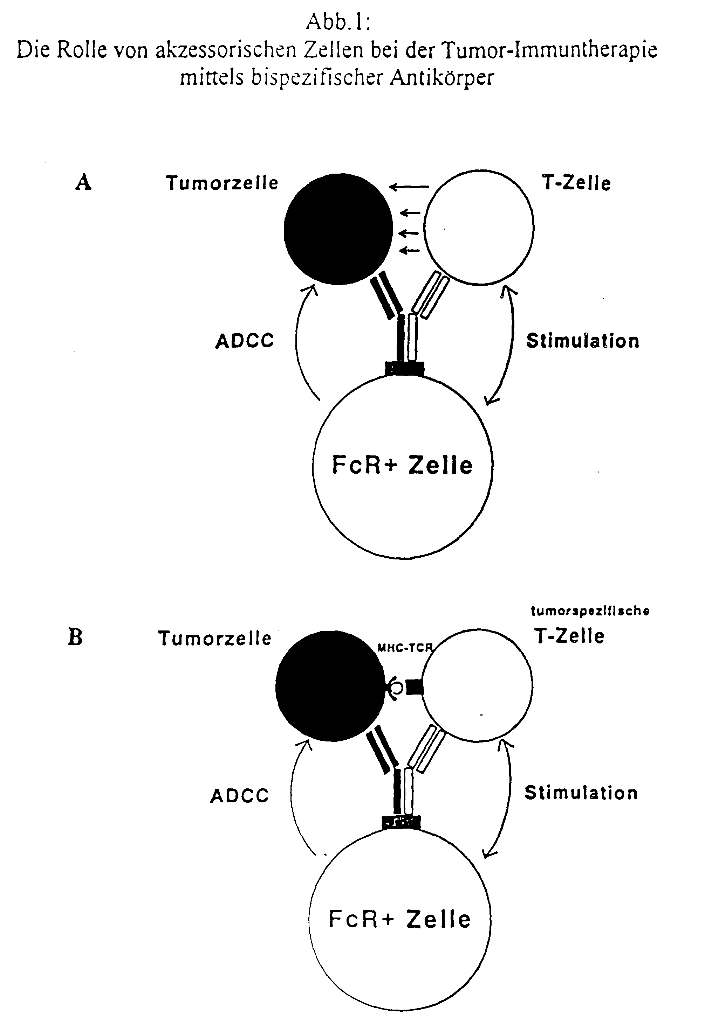

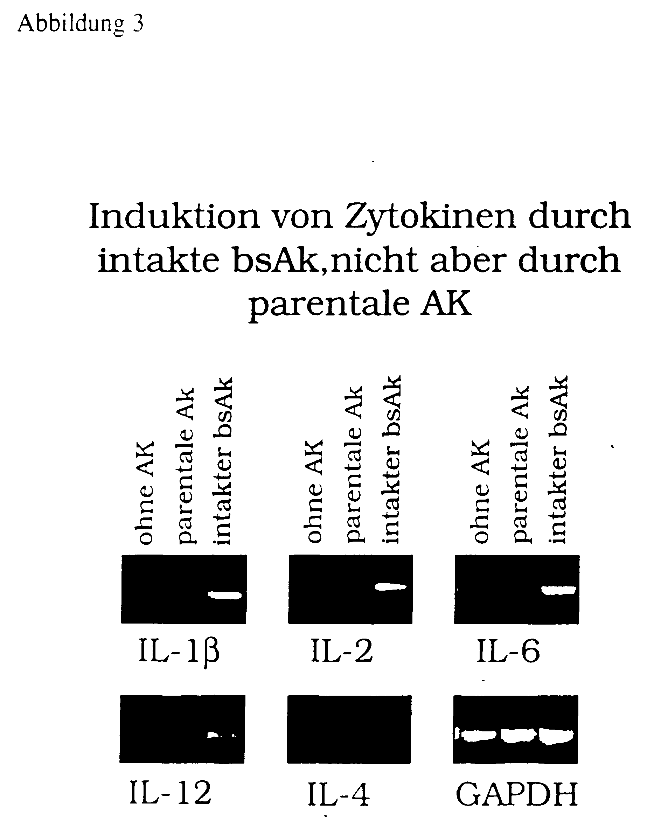

Auf der Tumorzelle erfolgt eine Hochregulation von MHC 1, sowie eine Aktivierung der intrazellulären Prozessierungsmaschinerie (Proteasom-Komplex) aufgrund der Freisetzung von Zytokinen (wie z.B. INF-γ und TNF-α) in unmittelbarer Nachbarschaft der Tumorzelle. Die Zytokine werden aufgrund bispezifischer Antikörpervermittelter Aktivierung von T-Zellen und akzessorischen Zellen freigesetzt (siehe Abb. 1 und 3). D.h. durch den intakten bsAk werden nicht nur Tumorzellen zerstört oder phagozytiert, sondern indirekt auch deren Tumorimmunität erhöht.

Die Aktivierung der Fc-Rezeptor positiven Zelle durch den bsAk ist von der Subklasse bzw. der Subklassenkombination des bsAk abhängig. Wie in in vitro-Versuchen nachgewiesen werden konnte, sind beispielsweise bsAk der Subklassenkombination Maus-IgG2a/Ratte-IgG2b in der Lage, an Fc-Rezeptor positive Zellen zu binden und diese gleichzeitig zu aktivieren, was zur Hochregulation bzw. Neuausbildung (Expression) von kostimulatorischen Antigenen, wie z.B. CD40, CD80 oder CD86, auf der Zelloberfläche dieser Zellen führt. Dagegen sind bsAk der Subklassenkombination Maus-IgG1/IgG2b zwar in der Lage an Fc-Rezeptor positive Zellen zu binden (1), können diese aber offenbar nicht in vergleichbarem Maße aktivieren (2).

Während der intakte bsAk die T-Zelle mit einem Bindungsarm (z.B. an CD3 oder CD2) bindet und gleichzeitig aktiviert, können kostimulatorische Signale von der, an den Fc-Teil des bsAk gebundenen Fc-Rezeptor positiven Zelle, an die T-Zelle übermittelt werden. D.h. erst die Kombination von Aktivierung der T-Zelle über einen Bindungsarm des bsAk und der gleichzeitigen Übertragung von kostimulatorischen Signalen von der Fc-Rezeptor positiven Zelle auf die T-Zelle, führt zu einer effizienten T-Zellaktivierung (Abb. 1A). Tumor-spezifische T-Zellen, die an der Tumorzelle ungenügend aktiviert wurden und anergisch sind, können nach der erfindungsgemäßen ex vivo-Vorbehandlung ebenfalls reaktiviert werden (Abb. 1B).

Ein weiterer wichtiger Aspekt bei der Induktion einer Tumorimmunität ist die mögliche Phagozytose, Prozessierung und Präsentation von Tumorebestandteilen durch die vom bsAk herangeführten und aktivierten akzessorischen Zellen (Monozyten/Makrophagen, dendritische Zellen und NK-"Natural Killer"-Zellen). Durch diesen klassischen Mechanismus der Präsentation von Antigenen können sowohl tumorspezifische CD4- wie auch CD8-positive Zellen generiert werden. Tumorspezifische CD4-Zellen spielen darüberhinaus eine wichtige Rolle für die Induktion einer humoralen Immunantwort im Zusammenhang mit der T-B-Zell Kooperation.

Bispezifische und trispezifische Antikörper können mit einem Bindungsarm an den T-Zellrezeptor-Komplex der T-Zelle, mit dem zweiten Bindungsarm an tumorassoziierte Antigene auf der Tumorzelle binden. Sie aktivieren dabei T-Zellen, die durch Freisetzung von Zytokinen oder Apoptose-vermittelnde Mechanismen die Tumorzellen zerstören. Darüber hinaus besteht offenbar die Möglichkeit, daß T-Zellen im Rahmen der Aktivierung mit bispezifischen Antikörpern tumorspezifische Antigene über ihren Rezeptor erkennen und dadurch eine dauerhafte Immunisierung eingeleitet wird (Abb. 1B). Von besonderer Bedeutung ist dabei der intakte Fc-Teil des bispezifischen oder trispezifischen Antikörpers, der die Bindung an akzessorische Zellen wie z.B. Monozyten/Makrophagen und dendritische Zellen vermittelt und diese veranlaßt selbst zytotoxisch zu werden und/oder gleichzeitig wichtige kostimulatorische Signale an die T-Zelle weiterzugeben (Abb. 1). Auf diese Weise kann offensichtlich eine T-Zellantwort u.U. auch gegen bislang unbekannte, tumorspezifische Peptide induziert werden.

Durch Redirektion von u.U. anergisierten, tumorspezifischen T-Zellen an Tumorzellen mittels bispezifischer und/oder trispezifischer Antikörper bei gleichzeitiger Kostimulation derartiger T-Zellen durch akzessorische Zellen welche an den Fc-Teil des bispezifischen oder trispezifische Antikörpers binden, könnte die Anergie von zytotoxischen T-Zellen (CTLs) aufgehoben werden. D.h. eine im Patienten gegen den Tumor existierende T-Zell-Toleranz kann mittels intakter heterologer bispezifischer und/oder trispezifischer Antikörper gebrochen und damit eine dauerhafte Tumorimmunität induziert werden.

Für den letzten Punkt gibt es erste in vivo-Daten aus Mausversuchen, die auf eine derartige dauerhafte Tumorimmuntät nach Behandlung mit einem syngenen Tumor und intakten bsAk hinweisen. In diesen Versuchen überlebten insgesamt 14 von 14 Tieren, die nach einer ersten Tumorinjektion erfolgreich mit bsAk behandelt werden konnten, eine weitere Tumorinjektion 144 Tage nach der ersten Tumorinjektion - ohne eine erneute Gabe von bsAk (siehe Beispiel 2).

Weitere Vorteile bei der Immunisierung durch die Applikation der mittels bispezifischer und/oder trispezifischer Antikörper hergestellten erfindungsgemäßen Tumorzellpräparate sind (i) die Minimierung von möglichen Nebenwirkungen, (ii) die kontrollierte Bindung des Tumorbindungsarms an die Tumorzelle außerhalb des Körpers und (iii) ein möglichst geringer Verbrauch von bispezifischen und trispezifischen Antikörpern. Dabei sind grundsätzlich zwei Vorgehensweisen möglich, die unten im einzelnen erläutert werden. Ein wichtiger Aspekt bei der Langzeitinkubation ist, daß der eingesetzte bispezifische oder trispezifische Antikörper innerhalb der projektierten Inkubationszeit verbraucht und abgebaut wird. Damit würde für eine derartige Immunisierung die langwierige Arzneimittelanmeldung entfallen.

Die Tumorzellen werden mit den Antikörpern beim Kurzeit- und beim Langzeit-Inkubationsverfahren über einen Zeitraum von 10 Minuten bis 5 Stunden, bevorzugt bis 3 Stunden, weiterhin bevorzugt bis 2 Stunden und noch weiter bevorzugt 15 Minuten bis 1 Stunde lang inkubiert. Die Inkubation erfolgt bevorzugt bei einer Temperatur von 4°C bis 25°C, insbesondere bevorzugt bei 4°C bis 10°C. Die Inkubation wird bevorzugt in steriler Umgebung in gepufferter Kochsalzlösung bei einem neutralen pH-Wert durchgeführt. Bei der Kurzzeit-Inkubation erfolgt anschließend unmittelbar die Reinfusion in den Patienten. Beim Langzeit-Inkubationsverfahren werden nach dieser Vorinkubation die mononukleären Zellen aus dem peripheren Blut zugegeben und zusammen mit den bereits vorinkubierten Tumorzellen/Antikörpern für einen weiteren Zeitraum von 1 bis 14 Tagen, bevorzugter 3 bis 10 Tagen, weiterhin bevorzugt 6 bis 10 Tagen, inkubiert. Diese Inkubation erfolgt bevorzugt bei 37°C unter sterilen Bedingungen und unter GMP-Bedingungen (Good Manufacturing Production = GMP) in einem Brutschrank. Wie weiter oben bereits beschrieben, können bei der Langzeit-Inkubation die Blutzellen auch zusammen mit den Tumorzellen und den Antikörpern unter geeigneten Bedingungen inkubiert werden.

Die obengenannten Inkubationsbedingungen sind nur beispielhaft zu verstehen. In Abhängigkeit von den Tumorzellen und den verwendeten Antikörpern können auch andere Zeiträume, Temperaturbedingungen etc., allgemein andere Inkubationsbedingungen, gewählt werden. Der Fachmann kann durch einfache Versuche diese Bedingungen festlegen.

Bei der Vorinkubation werden die Tumorzellen bevorzugt in einer Menge von 10⁷ bis 10⁹ Zellen, weiterhin bevorzugt in einer Menge von ca. 10⁸ Zellen, verwendet. Die mononukleären Zellen aus dem peripheren Blut werden bevorzugt in einer Menge von ca. 10⁸ bis 10¹⁰ Zellen zugegeben. Selbstverständlich ist es für den Fachmann auch möglich, andere Inkubationsbedingungen zu wählen, die durch Laborversuche ermittelt werden können (bspw. Änderungen in der Zellzahl und Inkubationsdauer).

Die eingesetzten autologen Tumorzellen werden, um nach Reinfusion ein weiteres Überleben der Tumorzellen zu verhindern, bspw. bestrahlt. Beispielsweise werden γ-Strahlen verwendet, die bspw. in einer Dosisstärke von 50 bis 100 Gy eingesetzt werden.

Die erfindungsgemäß eingesetzten Antikörper sind bevorzugt zur Reaktivierung von in Anergie befindlichen, tumorspezifischen T-Zellen befähigt. Weiterhin sind sie zur Induktion von tumorreaktiven Komplement-bindenden Antikörpern und damit zur Induktion einer humoralen Immunantwort in der Lage.

Die Bindung erfolgt bevorzugt über CD3, CD2, CD4, CD5, CD6, CD8, CD28 und/oder CD44 an die T-Zelle. Die Fc-Rezeptor positiven Zellen weisen zumindest einen Fcγ-Rezeptor I, II oder III auf.

Erfindungsgemäß einsetzbare Antikörper sind zur Bindung an Monozyten, Makrophagen, dendritische Zellen, "Natural Killer"-Zellen (NK-Zellen) und/oder aktivierte neutrophile Zellen als Fcγ-Rezeptor 1 positive Zellen befähigt.

Die erfindungsgemäß einsetzbaren Antikörper bewirken, daß die Expression von CD40, CD80, CD86, ICAM-1 und/oder LFA-3 als kostimulatorische Antigene oder/und die Sekretion von Zytokinen durch die Fc-Rezeptor positive Zelle initiiert oder erhöht wird. Die Zytokine sind bevorzugt IL-1, IL-2, IL-4, IL-6, IL-8,IL-12 und/oder TNF-α.

Die Bindung an die T-Zelle erfolgt bevorzugt über den T-ZellRezeptor-Komplex der T-Zelle.

Die erfindungsgemäß einsetzbaren bispezifischen Antikörper sind bevorzugt:
- ein anti-CD3 X anti-Tumor-assoziiertes Antigen- und/oder anti-CD4 X anti-Tumor-assoziiertes Antigen- und/oder anti-CD5 X anti-Tumor-assoziiertes Antigen- und/oder anti-CD6 X anti-Tumor-assoziiertes Antigen- und/oder anti-CD8 X anti-Tumor-assoziiertes Antigen- und/oder anti-CD2 X anti-Tumor-assoziiertes Antigen- und/oder anti-CD28 X anti-Tumor-assoziiertes Antigen- und/oder anti-CD44 X anti-Tumor-assoziiertes Antigen-Antikörper ist.

Die erfindungsgemäß einsetzbaren trispezifischen Antikörper sind bevorzugt:
- ein anti-CD3 X anti-Tumor-assoziiertes Antigen- und/oder anti-CD4 X anti-Tumor-assoziiertes Antigen- und/oder anti-CD5 X anti-Tumor-assoziiertes Antigen- und/oder anti-CD6 X anti-Tumor-assoziiertes Antigen- und/oder anti-CD8 X anti-Tumor-assoziiertes Antigen- und/oder anti-CD2 X anti-Tumor-assoziiertes Antigen- und/oder anti-CD28 X anti-Tumor-assoziiertes Antigen- und/oder anti-CD44 X anti-Tumor-assoziiertes Antigen-Antikörper.

Die erfindungsgemäß einsetzbaren trispezfischen Antikörper weisen zumindest eine T-Zell-Bindungsarm, einen Tumorzell-Bindungsarm und einen an Fc-Rezeptor positive Zellen bindenden Bindungsarm auf. Dieser zuletzt genannte Bindungsarm kann ein anti-Fc-Rezeptor-Bindungsarm oder ein Mannose-Rezeptor-Bindungsarm sein.

Der bispezifische Antikörper ist bevorzugt ein heterologer intakter Ratte/Maus bispezifischer Antikörper.

Mit den erfindungsgemäß einsetzbaren bispezifischen und trispezifischen Antikörpern werden T-Zellen aktiviert und gegen die Tumorzellen redirigiert. Bevorzugt einsetzbare heterologe intakte bispezifische Antikörper werden aus einer oder mehreren der nachfolgenden Isotyp-Kombinationen ausgewählt:
Ratte-IgG2b/Maus-IgG2a,
Ratte-IgG2b/Maus-IgG2b,
Ratte-IgG2b/Maus-IgG3,

Ratte-IgG2b/Human-IgG1,
Ratte-IgG2b/Human-IgG2,
Ratte-IgG2b/Human-IgG3[orientaler Allotyp G3m(st) = Bindung an Protein A],
Ratte-IgG2b/Human-IgG4,

Ratte-IgG2b/Ratte-IgG2c,
Maus-IgG2a/Human-IgG3[kaukasische Allotypen G3m(b+g) = keine Bindung an Protein A, im folgenden mit * gekennzeichnet]
Maus-IgG2a/Maus-[VH-CH1,VL-CL]-Human-IgG1-[Hinge]-Human-IgG3*-[CH2-CH3]
Maus-IgG2a/Ratte-[VH-CH1,VL-CL]-Human-IgG1-[Hinge]-Human-IgG3*-[CH2-CH3]
Maus-IgG2a/Human-[VH-CH1,VL-CL]-Human-IgG1-[Hinge]-Human-IgG3*-[CH2-CH3]
Maus-[VH-CH1,VL-CL]-Human-IgG1/Ratte-[VH-CH1,VL-CL]-Human-IgG1-[Hinge]-Human-IgG3*-[CH2-CH3]
Maus-[VH-CH1,VL-CL]-Human-IgG4/Ratte-[VH-CH1,VL-CL]-Human-IgG4-[Hinge]-Human-IgG4[N-terminale Region von CH2]-Human-IgG3*[C-terminale Region von CH2: > AS-Position 251]-Human- IgG3*[CH3]
Ratte-IgG2b/Maus-[VH-CH1,VL-CL]-Human-IgG1-[Hinge-CH2-CH3]
Ratte-IgG2b/Maus-[VH-CH1,VL-CL]-Human-IgG2-[Hinge-CH2-CH3]
Ratte-IgG2b/Maus-[VH-CH1,VL-CL]-Human-IgG3-[Hinge-CH2-CH3, orientaler Allotyp]
Ratte-IgG2b/Maus-[VH-CH1,VL-CL]-Human-IgG4-[Hinge-CH2-CH3]
Human-IgG1/Human-[VH-CH1,VL-CL]-Human-IgG1-[Hinge]-Human-IgG3*-[CH2-CH3]
Human-IgG1/Ratte-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]
Human-IgG1/Maus-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]
Human-IgG1/Ratte-[VH-CH1,VL-CL]-Human-IgG1-[Hinge]-Human-IgG2[N-terminale Region von CH2]-Human-IgG3*[C-terminale Region von CH2 : > AS-Position 251]-Human-IgG3*[CH3]
Human-IgG1/Maus-[VH-CH1,VL-CL]-Human-IgGl-[Hinge]-Human-IgG2[N-terminale Region von CH2]-Human-IgG3*[C-terminale Region von CH2 : > AS-Position 251]-Human-IgG3*[CH3]
Human-IgG1/Ratte-[VH-CH1,VL-CL]-Human-IgG1-[Hinge]-Human-IgG3*-[CH2-CH3]
Human-IgG1/Maus-[VH-CH1,VL-CL]-Human-IgG1-[Hinge]-Human-IgG3*-[CH2-CH3]
Human-IgG2/Human-[VH-CH1,VL-CL]-Human-IgG2-[Hinge]-Human-IgG3*-[CH2-CH3]
Human-IgG4/Human-[VH-CH1,VL-CL]-Human-IgG4-[Hinge]-Human-IgG3*-[CH2-CH3]
Human-IgG4/Human-[VH-CH1,VL-CL]-Human-IgG4-[Hinge]-Human-IgG4[N-terminale Region von CH2]-Human-IgG3*[C-terminale Region von CH2 : > AS-Position 251]-Human-IgG3*[CH3]
Maus-IgG2b/Ratte-[VH-CH1,VL-CL]-Human-IgG1-[Hinge]-Human-IgG3*-[CH2-CH3]
Maus-IgG2b/Human-[VH-CH1,VL-CL]-Human-IgG1-[Hinge]-Human-IgG3*-[CH2-CH3]
Maus-IgG2b/Maus-[VH-CH1,VL-CL]-Human-IgG1-[Hinge]-Human-IgG3*-[CH2-CH3]
Maus-[VH-CH1,VL-CL]-Human-IgG4/Ratte-[VH-CH1,VL-CL]-Human-IgG4-[Hinge]-Human-IgG4-[CH2]-Human-IgG3*-[CH3]
HumanIgG1/Ratte[VH-CH1,VL-CL]-Human-IgG1[Hinge]-Human-IgG4-[CH2]-HumanIgG3*[CH3]
HumanIgG1/Maus[VH-CH1,VL-CL]-Human-IgG1[Hinge]-Human-IgG4-[CH2]-Human-IgG3*[CH3]
Human-IgG4/Human[VH-CH1,VL-CL]-Human-IgG4-[Hinge]-Human-IgG4-[CH2]-HumanIgG3*-[CH3].

Die oben genannten Antikörper sind beispielhaft genannt. Diese und andere erfindungsgemäß einsetzbaren Antikörper können vom Fachmann anhand von speziellen Techniken hergestellt werden. Beispielhaft hierfür sind die Literaturstellen (7) bis (11) genannt.

Insbesondere Greenwood et. al. beschreiben die Möglichkeit, einzelne Immunglobulin-Domänen (z.B. CH2) durch geeignete Klonierungstechniken auszutauschen. Dadurch wird die Technik bereitgestellt, um neue Antikörperkombinationen herzustellen, wie beispielhaft in Anspruch 9 beschrieben, z.B.: human-(VH-CH1, VL-CL)-human IgG4-(hinge)-human IgG4 (N-terminale Region von CH2)-human IgG3* (C-terminale Region von CH2:> Aminosäureposition 251)-human IgG3* (CH3).

Die Kombination mit einem Antikörper: human IgG4 zur Herstellung des bispezifischen Antikörpers: human IgG4/human-(VH-CH1, VL-CL)-human IgG4-(hinge)-human IgG4 (N-terminale Region von CH2)-human IgG3* (C-terminale Region von CH2:> Aminosäureposition 251)- human IgG3* (CH3) wird durch einfache Zellfusion, wie in Lindhofer et al. (J. Immunol. 155:219, 1995) beschrieben, herbeigeführt.

Bei den erfindungsgemäß verwendbaren Antikörpern handelt es sich vorzugsweise um monoklonale, chimäre, rekombinante, synthetische, halbsynthetische oder chemisch modifizierte intakte Antikörper mit beispielsweise Fv-, Fab-, scFv- oder F(ab)₂-Fragmenten.

Bevorzugt werden Antikörper oder Derivate oder Fragmente vom Menschen verwendet oder solche, die derart verändert sind, daß sie sich für die Anwendung beim Menschen eignen (sogenannte "humanized antibodies") (siehe z.B. Shalaby et al., J. Exp. Med. 175 (1992), 217; Mocikat et al., Transplantation 57 (1994), 405).

Die Herstellung der verschiedenen oben genannten Antikörpertypen und -fragmente ist dem Fachmann geläufig. Die Herstellung monoklonaler Antikörper, die ihren Ursprung vorzugsweise in Säugetieren, z.B. Mensch, Ratte, Maus, Kaninchen oder Ziege, haben, kann mittels herkömmlicher Methoden erfolgen, wie sie z.B. in Köhler und Milstein (Nature 256 (1975), 495), in Harlow und Lane (Antibodies, A Laboratory Manual (1988), Cold Spring Harbor) oder in Galfré (Meth. Enzymol. 73 (1981), 3) beschrieben sind.

Ferner ist es möglich, die beschriebenen Antikörper mittels rekombinanter DNA-Technologie nach dem Fachmann geläufigen Techniken herzustellen (siehe Kurucz et al., J. Immunol. 154 (1995), 4576; Hollinger et al., Proc. Natl. Acad. Sc. USA 90 (1993), 6444).

Die Herstellung von Antikörpern mit zwei verschiedenen Spezifitäten, den sogenannten bispezifischen Antikörpern, ist zum einen durch Anwendung rekombinanter DNA-Technologie möglich, aber auch durch die sogenannte Hybrid-Hybridoma-Fusionstechnik (siehe z.B. Milstein et al., Nature 305 (1983), 537). Hierbei werden Hybridomazellinien, die Antikörper mit jeweils einer der gewünschten Spezifitäten produzieren, fusioniert und rekombinante Zellinien identifiziert und isoliert, die Antikörper mit beiden Spezifitäten produzieren.

Das der Erfindung zugrunde liegende Problem kann sowohl durch bispezifische als auch trispezifische Antikörper gelöst werden, sofern sie die im Anspruch 1 gekennzeichneten Eigenschaften und Wirkungen aufweisen. Nachfolgend wird die Herstellung von Antikörpern mit Zwei- und Dreispezifitäten näher beschrieben. Die Bereitstellung derartiger bispezifischer und trispezifischer Antikörper gehört zum Stand der Technik, und auf die derartige Herstellungstechniken beschreibende Literatur wird hier voll inhaltlich Bezug genommen.

Die Herstellung von Antikörpern mit drei Spezifitäten, sogenannten trispezifischen Antikörpern, durch die das der Erfindung zugrundeliegende Problem ebenfalls lösbar ist, kann beispielsweise derart erfolgen, daß an eine der schweren Ig-Ketten eines bispezifischen Antikörpers eine dritte Antigenbindungsstelle mit einer weiteren Spezifität, z.B. in Form eines "single chain variable fragments" (scFv), angekoppelt wird. Das scFv kann beispielsweise über einen
-S-S(G₄S)ₙD-I-Linker an eine der schweren Ketten gebunden sein (S = Serin, G = Glycin, D = Aspartat, I = Isoleucin).

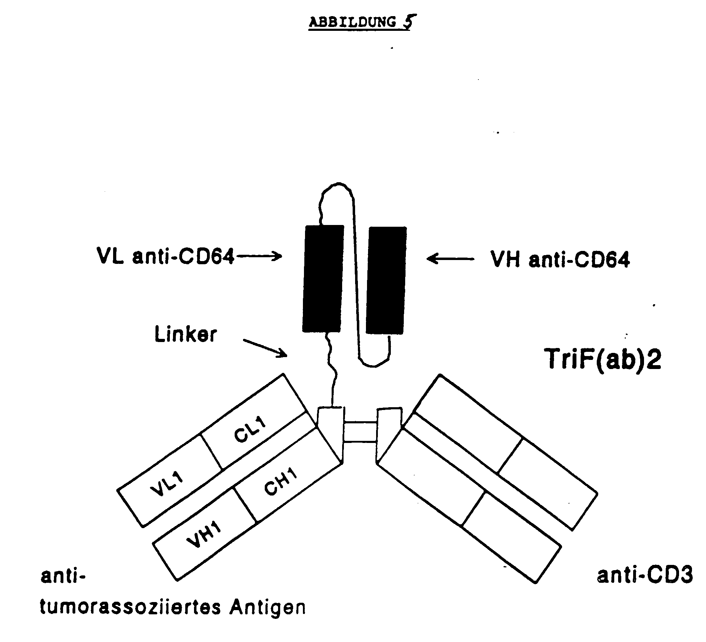

Analog dazu können trispezifische F(ab)₂-Konstrukte hergestellt werden, indem die CH2-CH3-Regionen der schweren Kette einer Spezifität eines bispezifischen Antikörpers ersetzt werden durch ein scFv mit einer dritten Spezifität, während die CH2-CH3-Regionen der schweren Kette der anderen Spezifität beispielsweise durch Einbau eines Stopcodons (am Ende der "Hinge"-Region) in das codierende Gen, z.B. mittels homologer Rekombination, entfernt werden (siehe Abb.5).

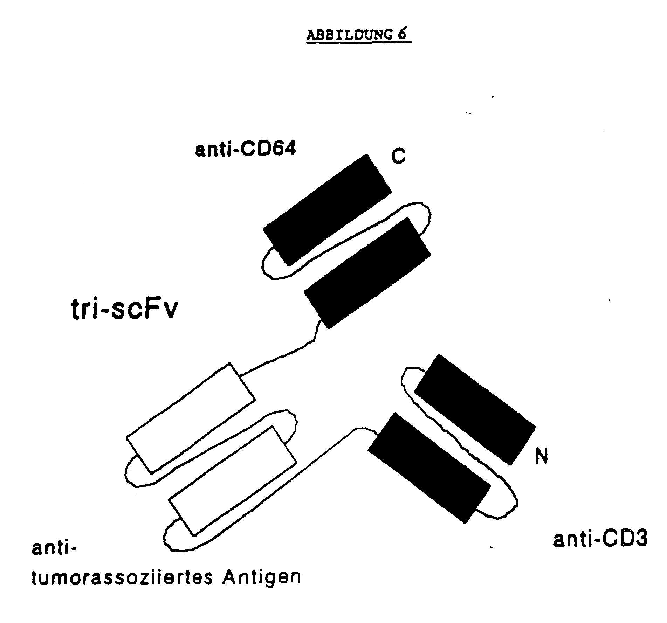

Möglich ist auch die Herstellung trispezifischer scFv-Konstrukte. Hierbei werden drei VH-VL-Regionen, die drei verschiedene Spezifitäten repräsentieren, hintereinander in Reihe angeordnet (Abb.6).

Erfindungsgemäß werden z.B. intakte bispezifische Antikörper verwendet. Intakte bispezifische Antikörper sind aus zwei Antikörper-Halbmolekülen (je eine H- und L-Immunglobulinkette), die jeweils eine Spezifität repräsentieren, zusammengesetzt, und besitzen darüber hinaus, wie normale Antikörper auch, einen Fc-Teil mit den bekannten Effektorfunktionen. Sie werden bevorzugt durch die Quadrom-Technologie hergestellt. Dieses Herstellungsverfahren ist beispielhaft in der DE-A-44 19 399 beschrieben. Auf diese Druckschrift, auch bzgl. einer Definition der bispezifischen Antikörper, wird zur vollständigen Offenbarung vollinhaltlich Bezug genommen. Selbstverständlich sind auch andere Herstellungsverfahren einsetzbar, solange sie zu den erfindungsgemäß notwendigen intakten bispezifischen Antikörpern der obigen Definition führen.

Beispielsweise können in einem neu entwickelten Herstellungsverfahren (6) intakte bispezifische Antikörper in ausreichender Menge produziert werden. Die Kombination von 2 bispezifischen Antikörpern gegen 2 unterschiedliche tumorassoziierte Antigene (z.B. c-erb-B2, ep-cam, beispielsweise GA-733-2 = C215) auf den Mammakarzinomzellen minimiert die Gefahr, daß Tumorzellen, die nur ein Antigen exprimieren, unerkannt bleiben.

Es wurde auch versucht, durch Behandlung mit bispezifischen F(ab')2-Fragmenten mit den Spezifitäten anti-c-erb-B2 x antiCD64 eine Tumorimmunität zu erreichen. Der Hauptnachteil von bsF(ab')2-Fragmenten liegt darin, daß aufgrund der verwendeten Spezifitäten lediglich FcγRI+ Zellen an den Tumor redirigiert werden. T-Zellen werden durch diesen bispezifischen Antikörper nicht an den Tumor redirigiert. Die bsF(ab')2-Fragmente besitzen zwar auch das Potential zur direkten Tumorzerstörung, sind aber nicht in der Lage, selber eine Tumorimmunität zu etablieren. Dazu ist nur die T-Zelle mit ihrem spezifischen T-Zellrezeptor befähigt. Die FcγRI+ Zellen können zwar indirekt durch Präsentation von tumorspezifischen Peptiden (über MHC I bzw. MHCII) nach z.B. Phagozytose von Tumorzellbestandteilen tumorspezifische T-Zellen aktivieren, die Effizienz der Induktion einer Tumorimmunität ist hier aber nicht ganz so hoch (nur bei 30% der Patienten).
Weitere Vorteile von intakten bsAk mit der Fähigkeit zur Redirektion von T-Zellen gegenüber den o.g. bsF(ab')2 Fragmenten sind im einzelnen:
1. An intakte bsAk können Fc-Rezeptor positive Zellen binden und einerseits über ADCC (antibody-dependent cell-mediated cytotoxicity) direkt zur Tumorzerstörung beitragen sowie andererseits wie oben näher ausgeführt zur T-Zellaktivierung.
2. Durch intakte T-Zell-redirigierende bsAk werden auch anergisierte tumorspezifische T-Zellen an die Tumorzelle herangeführt, die erfindungsgemäß direkt am Tumor reaktiviert werden können. Dies kann durch ein bsF(ab')2-Fragment mit den Spezifitäten anti-CD64Xanti-tumorassoziiertes Antigen nicht erreicht werden.
3. Ein bsF(ab')2-Fragment mit den Spezifitäten anti-CD64Xanti-tumorassoziiertes Antigen kann lediglich eine Tumorimmunität in 30% der Patienten erzielen, während erfindungsgemäß in Mausversuchen mit T-Zell-redirigierenden intakten bsAk ein Schutz in 100% der Tiere erzielt werden konnte.

Die Bindung des bsAk an Fcγ-RI besitzt zwei wesentliche Vorteile im Hinblick auf eine optimale anti-Tumorwirksamkeit:
(1) Fcγ-RI positive Zellen besitzen die Fähigkeit mittels ADCC Tumorzellen zu eliminieren (11) und können insofern synergistisch zur anti-Tumorwirkung der durch den bsAk an die Tumorzelle herangeführten cytotoxischen T-Zellen beitragen (13).
(2) Fcγ-RI positive Zellen (wie z.B. Monozyten/Makrophagen/Dendriten) sind in der Lage, wichtige kostimulatorische Signale, ähnlich wie bei der Antigen-Präsentation, der T-Zelle zu liefern und damit eine Anergisierung der T-Zelle zu verhindern. Wie in Abbildung 1 gezeigt können weiterhin, als erwünschtes Nebenprodukt, aufgrund der durch intakten bsAk vermittelten Interaktion von T-Zelle mit akzessorischer Zelle und Tumorzelle sogar T-Zellen, deren T-Zellrezeptor tumorspezifische Peptide (über MHC Antigene auf der Tumorzelle präsentiert) erkennt, stimuliert werden. Die für eine korrekte Aktivierung der T-Zelle notwendigen Kostimuli würden in dieser Konstellation von der akzessorischen Zelle (z.B. Monocyt) geliefert werden. Insofern sollte der erfindungsgemäße Antikörper neben der direkten T-Zellrezeptor-unabhängigen, durch bsAk vermittelten Tumorzerstörung (Abb.1A) ebenfalls tumorspezifische T-Zellen aktivieren und generieren (Abb.1B), die nach Abbau der bsAk weiterhin im Patienten patrouillieren. D.h. mittels intakter bsAk kann ähnlich wie bei gentherapeutischen Ansätzen (z.B. durch Einbau von kostimulatorischen Antigenen wie B-7 in die Tumorzelle) die Tumortoleranz im Patienten durchbrochen werden.

Günstig in diesem Zusammenhang ist weiterhin, daß die Expression von Fcγ-RI nach G-CSF -Behandlung auf den entsprechenden Zellen hochreguliert wird.

Die Erfindung wurde vorstehend und wird nachstehend insbesondere anhand von bispezifischen Antikörpern beschrieben. An die Stelle der bispezifischen Antikörper sind selbstverständlich aber auch trispezifische Antikörper einsetzbar, solange sie die gestellten Bedingungen erfüllen.

Die Erfindung wurde und wird anhand der beiliegenden Abbildungen näher beschrieben. Die Abbildungen zeigen:
- Abb. 1:: Die Rolle akzessorischer Zellen bei der Tumor-Immuntherapie mittels bispezifischer Antikörper
- Abb. 2:: Zerstörung von Tumorzellen nach Gabe von bispezifischen Antikörpern, nachgewiesen im Durchflußzytometer
- Abb. 3:: Induktion von Zytokinen durch intakte bispezifische Antikörper nur nicht aber durch parentale Antikörper
- Abb. 4:: Wirksamkeit des erfindungsgemäßen Verfahrens in vivo
- Abb. 5:: Trispezifische F(ab)₂-Antikörper
- Abb. 6:: Trispezifischer scFv-Antikörper

### IMMUNISIERUNGSPROTOKOLLE

### Ex vivo-Immunisierung (Kurzzeitinkubation)

1. Herstellung einer Einzelzellsuspension (10⁷-10⁹ Zellen) aus autologem Tumormaterial (oder allogenen Tumrozellen desselben Tumortyps) mit anschließender γ-Bestrahlung (50-100 Gy)
2. Zugabe von bsAk (5-50 µg) und 45 minütige Inkubation bei 4°C. Anschließend Auswaschen von ungebundenem Antikörper
3. Reinfusion des Zellgemisches (i.v.)

### Ex vivo-Immunisierung (Langzeitinkubation)

1. Herstellung einer Einzelzellsuspension (10⁷-10⁹ Zellen) aus autologem Tumormaterial (oder allogenen Tumorzellen desselben Tumortyps) mit anschließender γ-Bestrahlung (50-100 Gy)
2. Zugabe von bsAk (5-50 µg), 45 min inkubieren
3. Zugabe von PBMC (10⁸-10¹⁰), [alternativ: 1 x 10⁹ Zellen aus T-Zellapherese]
4. nach 5 bis 7 Tagen Kontrolle der T-Zell-Reaktivität durch Transfer von Aliquots auf z.B. allogene Brustkrebszellinien (MCF-7, MX-1)
5. Reinfusion (i.v.) der kultivierten PBMC am Tag 4 bis 14 in den Patienten (bei T-Zellapherese Kryokonservierung)

Abkürzungen: PBMC, peripheral blood mononucleated cells = mononukleäre Zellen aus dem peripheren Blut; i.v., intravenös.

Ein ähnlicher aber auf den Zusatz von Zytokinen angewiesener und mit konventionellen bsAk (keine Aktivierung von akzessorischen Zellen durch bsAk der Subklassenkombination Ratte IgG2B X Ratte IgG1) durchgeführter Ansatz zeigte die prinzipielle Wirksamkeit einer derartigen ex vivo-Immunisierung im Tiermodell (5).

Im Gegensatz dazu liegt der Vorteil des hier offengelegten Verfahrens in der "Selbstversorgung" mit den für die Hochregulation von z.B. MHC 1 auf der Tumorzelle benötigten Zytokinen (wie INF-γ oder TNF-α) durch die gleichzeitige Aktivierung von T-Zellen und akzessorischen Zellen (Monozyten/Makrophagen, Abb.) an der Tumorzelle. Dies wird durch die eingangs erwähnte besondere Subklassenkombination des hier verwendeten intakten bsAk erreicht. Bei der Kurzzeitinkubation finden diese Vorgänge im Patienten statt. Weitere Vorteile bei der Kurzzeitinkubation sind (i) Umgehung der sonst notwendigen Kultivierung der Zellsuspension mit serumhaltigen Medium. (ii) Damit entfällt auch die kostenintensive Kultivierung unter GMP-Richtlinien. (iii) Ein weiterer wichtiger Aspekt ist die Vermeidung bzw. Reduzierung von möglichen Nebenwirkungen durch den bsAk aufgrund der wesentlich geringeren Menge an appliziertem Antikörper.

Vorteil bei der Langzeitinkubation ist, daß der bsAk sich in vitro nach einiger Zeit selber verbraucht (und somit auch diese Methode nicht als Medikament, sondern als "medical device" etabliert werden kann).

### BEISPIEL 1

### Bispezifische Antikörper-vermittelte Lyse von Tumorzellen durch allogene T-Zellen

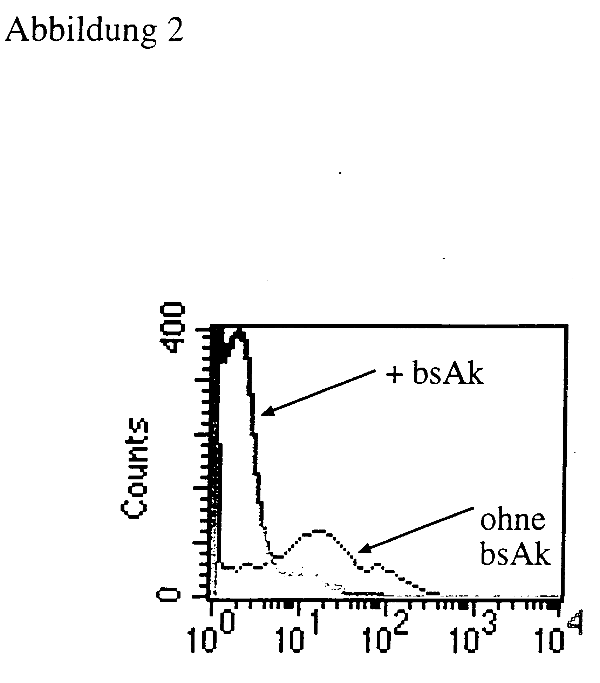

H-LAc78 ist eine Zellinie, die aus einem Hypopharynxkarzinom etabliert wurde und epcam in hohem Maße exprimiert (eigene FACS-Daten). Unter Verwendung von H-Lac78 und peripheren, mononukleären Zellen (PBMC) aus freiwilligen Spendern konnte man die Generierung allogener zytotoxischer T-Lymphozyten nachweisen. Hierzu wurden konstante Mengen H-Lac78 (2 x 10⁴) mit unterschiedlichen Mengen PBMCs in Gegenwart (10 ng) oder Abwesenheit eines bsAk (anti-epcam X anti-CD3) inkubiert. Nach einem Zeitraum von sieben Tagen wurden die PBMCs abgenommen und im Durchflußzytometer analysiert. Gleichzeitig wurden die Zahl der H-Lac78 Tumorzellen bestimmt. Die Aktivierung der T-Zellen läßt sich mikroskopisch an der Clusterbildung beobachten, die Proliferation kann anhand des Einbaus von radioaktiven Thymidin bestätigt werden. Der Nachweis verbliebener Tumorzellen wird mikroskopisch sowie über den epithelialen Marker epcam geführt, der auf Zellen des peripheren Blutes nicht exprimiert wird. Wie in Abb. 2 gezeigt, wurden die H-Lac78-Zellen bei Anwesenheit des bsAk vollständig lysiert, d.h. im Durchflußzytometer waren nach sieben Tagen keine epcam positiven Zellen mehr nachweisbar. Diese Daten wurden durch den mikroskopischen Eindruck bestätigt. Ohne bsAk war dagegen ein vollständiger Rasen von H-Lac78-Zellen in den Wells zu sehen und im FACS waren epcam positive Zellen nachweisbar.

### Nachweis aktivierter allospezifischer CTLs per Transferexperiment

In einem anschließenden Transferexperiment wurden die mit bzw. ohne bsAk inkubierten PBMCs auf neue H-Lac78-Zellen ohne erneute Zugabe von bsAk überführt. Auch hier wurden die Tumorzellen lysiert, und zwar ausschließlich von den zuvor durch den bsAk aktivierten PBMC. Die Lyse von H-Lac78 vollzog sich vollständig binnen 24 Stunden bis zu einem Verhältnis von 2 PBMC zu 1 H-Lac78-Zellen. Dieses Resultat bedeutet die Generierung allospezifischer CTLs ohne die externe Zugabe von Interleukin-2 (IL-2). Da IL-2 für die Aktivierung von T-Lymphozyten essentiell ist, sprechen die hier vorgelegten Daten dafür, daß durch die bsAk vermittelte Aktivierung IL-2 von den T-Zellen selbst produziert wird. Die Induktion der IL-2 mRNA bei Zugabe des bsAk konnte anschließend durch RT-PCR bestätigt werden, wobei der bsAk den parentalen Ausgangsantikörpern deutlich überlegen war (Abb. 3). Diese Beobachtung ist insofern von Bedeutung, als IL-2 zwar als antitumoral wirksames Zytokin beschrieben wurde, eine systemische Gabe in entsprechender Konzentration wegen seiner Toxizität jedoch nur bedingt möglich ist. Die Gefahr der Toxizität ist hingegen bei der lokalen Produktion von IL-2, wie sie beispielsweise durch intakte bsAk induziert wird, nicht gegeben. Da für die effektive Induktion von IL-2 (und IL-12) eine Stimulation von T-Zellen über den T-Zell-Rezeptor und CD28 notwendig ist, läßt auch dies auf die Bedeutung der Fc-Rezeptor positiven Zellen (welche die Liganden für CD28: CD80 und CD86 bereitstellen) bei der Aktivierung der T-Zellen mittels intakter bsAk schließen.

### BEISPIEL 2

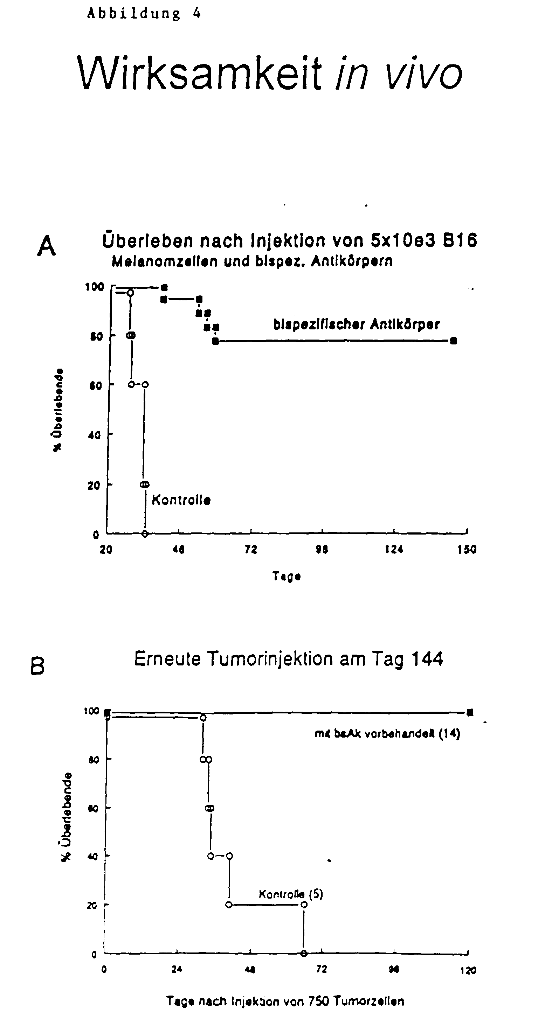

Zur Prüfung der Frage, ob bispezifische Antikörper eine langandauernde Tumorimmunität induzieren können, wurden C57BL/6 Mäusen zunächst 5 x 10³ syngene B16 Tumorzellen injiziert. Zwei Tage später wurde eine Gruppe von Mäusen (Anzahl 18) mit intaktem, mittels Quadrom-Technologie (6) hergestelltem, bsAk behandelt, welcher eine Zielstruktur (ep-cam/C215 = tumorassoziiertes Antigen) auf der Tumorzelle sowie CD3 auf T-Zellen erkennt. Eine zweite Gruppe (Anzahl 6) erhielt lediglich eine äquimolare Menge von Fab-Fragmenten der beiden im bsAk enthaltenen Spezifitäten. Während alle Tiere der Fab-Kontrollgruppe innerhalb von 56 Tagen verstarben oder eingeschläfert werden mußten, überlebten 14 von 18 mit bsAk behandelten Tiere. 144 Tage nach der ersten Injektion von Tumorzellen wurde den überlebenden 14 Tieren erneut eine Dosis von 750 B16 Tumorzellen, diesmal ohne Gabe von bsAk, injiziert. Zur Kontrolle wurde 5 unbehandelten Tieren dieselbe Tumorzellzahl verabreicht. Während das letzte Tier der unbehandelten Kontrollgruppe 66 Tage nach Tumorinjektion eingeschläfert werden mußte, überlebten alle mit bsAk behandelten Tiere (Überwachungszeitraum: 120 Tage nach zweiter Tumorzell-Injektion). Siehe auch Abbildung 4A und B: Überlebenskurven der beiden aufeinanderfolgenden, oben beschriebenen, Experimente.

### LITERATUR

1. Haagen et al., Interaction of human moncyte Fcγ receptors with rat IgG2b, J. Immunolog., 1995, 154: 1852-1860
2. Gast G.C., Haagen I.-A., van Houten A.A., Klein S., Duits A.J., de Weger R.A., Vroom T.M., Clark M.R., J. Phillips, van Dijk A.J.G., de Lau W.B.M., Bast B.J.E.G. CD8 T-cell activation after intravenous administration of CE3 X CD19 bispecific antibody in patients with non-Hodgkin lymphoma. Cancer Immunol. Immunother. 40: 390, 1995
3. Tenny, C., Jacobs, S., Stoller, R., Earle, M., and Kirkwood, J. Adoptive cellualr immunotherapy with high-dose chemotherapy and autologous bone marrow rescue (ABMR) for recurrent breast cancer (meeting abstract). Proc.Annu.Meet.Am.Soc.Clin.Oncol; 11: A88, 1992 ISSN: 0736-7589. CO: PMAODO - 7589 CO, 1993.
4. Early Breast Cancer Trialists' Collaborative Group, Systemic treatment of early breast cancer by hormonal, cytotoxic, or immune therapy - 133 randomised trials involving 31 000 recurrences and 24 000 deaths among 75 000 women. Part II Lancet 339: 71-85, 1992
5. Guo et al., Effective tumor vaccines generated by in vitro modification ot tumor cells with cytokines and bispecific monoclonal antibodies. Nature Medicine 3: 451, 1997
6. Lindhofer et al., Preferential species-restricted heavy-light chain pairing in rat-mouse quadromas: Implications for a single step purification of bispecific antibodies, J. Immunology 1995, 155:219
7. Brüggemann et al., A MATCHED SET OF RAT/MOUSE CHIMERIC ANTIBODIES: Identification and Biological Properties of Rat H Chain Constant Regions µ, γ1, γ2a, γ2b, ε, and α¹, J. Immunology 1989, 142:3145.
8. Routledge et al., A humanized monovalent CD3 antibody which can activate homologous complement, Eur. J. Immunology 1991, 21:2717
9. Greenwood et al., Structural motifs involved in human IgG antibody effector functions, Eur. J. Immunology 1993, 23:1098
10. Kardinal et al., Genetic stability of gene targeted immunoglobolin loci. I. Heavy chain isotype exchange induced by a unhiversal gene replacement vector, J. Immunology 1996, 89:309
11. Kardinal et al., Integration vectors for antibody chimerization by homologous recombination in hybridoma cells, Eur. J. Immunology 1995, 25:792.

## Patentansprüche

1. Verfahren zur Herstellung eines Tumorzellpräparats zur Immunisierung von Mensch und Tier mit den nachfolgenden Schritten:
a) Isolierung autologer Tumorzellen;
b) Behandeln der Tumorzellen, um ihr Überleben nach Reinfusion zu verhindern;
c) Inkubation der so behandelten Tumorzellen mit intakten heterologen bispezifischen und/oder trispezifischen Antikörpern, die die nachfolgenden Eigenschaften aufweisen:
α - Binden an eine T-Zelle;
β - Binden an zumindest ein Antigen auf einer Tumorzelle;
γ - Binden durch ihren Fc-Teil (bei bispezifischen Antikörpern) oder durch eine dritte Spezifität (bei trispezifischen Antikörpern) an Fc-Rezeptor positive Zellen.

2. Verfahren nach Anspruch 1,
dadurch gekennzeichnet, daß
die Antikörper so ausgewählt werden, daß sie zur Bindung Fc-Rezeptor positive Zellen befähigt sind, die einen Fcγ-Rezeptor I, II oder III aufweisen.

3. Verfahren nach Anspruch 2,
dadurch gekennzeichnet, daß
die Antikörper zur Bindung an Monozyten, Makrophagen, dendritische Zellen, "Natural Killer"-Zellen (NK-Zellen) und/oder aktivierte neutrophile Zellen als Fcγ-Rezeptor I positive Zellen befähigt sind.

4. Verfahren nach einem oder mehreren der vorhergehenden Ansprüche,
dadurch gekennzeichnet, daß
die Antikörper zur Induktion von tumorreaktiven Komplement-bindenden Antikörpern und damit zur Induktion einer humoralen Immunantwort befähigt sind.

5. Verfahren nach einem oder mehreren der vorhergehenden Ansprüche,
dadurch gekennzeichnet, daß
die Antikörper so ausgewählt werden, daß sie über CD2, CD3, CD4, CD5, CD6, CD8, CD28 und/oder CD44 an die T-Zellen binden.

6. Verfahren nach einem oder mehreren der vorhergehenden Ansprüche,
dadurch gekennzeichnet, daß
die Antikörper so ausgewählt werden, daß nach ihrer Bindung an die Fc-Rezeptor positiven Zellen die Expression von CD40, CD80, CD86, ICAM-1 und/oder LFA-3 als kostimulatorische Antigene und/oder die Sekretion von Zytokinen durch die Fc-Rezeptor positive Zelle initiiert oder erhöht wird.

7. Verfahren nach Anspruch 6,
dadurch gekennzeichnet, daß
die Antikörper so ausgewählt werden, daß die Sekretion von IL-1, IL-2, IL-4, IL-6, IL-8, IL-12 als Zytokine und/oder von TNF-α erhöht wird.

8. Verfahren nach einem oder mehreren der vorhergehenden Ansprüche,
dadurch gekennzeichnet, daß
der bispezifische Antikörper so ausgewählt wird, daß er ein anti-CD3 X anti-Tumor-assoziiertes Antigen- und/oder anti-CD4 X anti-Tumor-assoziiertes Antigen- und/oder anti-CD5 X anti-Tumor-assoziiertes Antigen- und/oder anti-CD6 X anti-Tumor-assoziiertes Antigen- und/oder anti-CD8 X anti-Tumor-assoziiertes Antigen- und/oder anti-CD2 X anti-Tumor-assoziiertes Antigen- und/oder anti-CD28 X anti-Tumor-assoziiertes Antigen- und/oder anti-CD44 X anti-Tumor-assoziiertes Antigen-Antikörper ist.

9. Verfahren nach einem oder mehreren der vorhergehenden Ansprüche,
dadurch gekennzeichnet, daß
der bispezifische Antikörper aus einer oder mehreren der nachfolgenden Isotykombinationen ausgewählt wird:
Ratte-IgG2b/Maus-IgG2a,
Ratte-IgG2b/Maus-IgG2b,
Ratte-IgG2b/Maus-IgG3,
Ratte-IgG2b/Human-IgG1,
Ratte-IgG2b/Human-IgG2,
Ratte-IgG2b/Human-IgG3[orientaler Allotyp G3m(st) = Bindung an Protein A],
Ratte-IgG2b/Human-IgG4,
Ratte-IgG2b/Ratte-IgG2c,
Maus-IgG2a/Human-IgG3[kaukasische Allotypen G3m(b+g) = keine Bindung an Protein A, im folgenden mit * gekennzeichnet]
Maus-IgG2a/Maus-[VH-CH1,VL-CL]-Human-IgG1-[Hinge]-Human-IgG3*-[CH2-CH3]
Maus-IgG2a/Ratte-[VH-CH1,VL-CL]-Human-IgG1-[Hinge]-Human-IgG3*-[CH2-CH3]
Maus-IgG2a/Human-[VH-CH1,VL-CL]-Human-IgG1-[Hinge]-Human-IgG3*-[CH2-CH3]
Maus-[VH-CH1,VL-CL]-Human-IgG1/Ratte-[VH-CH1,VL-CL]-Human-IgG1-[Hinge]-Human-IgG3*-[CH2-CH3]
Maus-[VH-CH1,VL-CL]-Human-IgG4/Ratte-[VH-CH1,VL-CL]-Human-IgG4-[Hinge]-Human-IgG4[N-terminale Region von CH2]-Human-IgG3*[C-terminale Region von CH2: > AS-Position 251]-Human- IgG3*[CH3]
Ratte-IgG2b/Maus-[VH-CH1,VL-CL]-Human-IgG1-[Hinge-CH2-CH3]
Ratte-IgG2b/Maus-[VH-CH1,VL-CL]-Human-IgG2-[Hinge-CH2-CH3]
Ratte-IgG2b/Maus-[VH-CH1,VL-CL]-Human-IgG3-[Hinge-CH2-CH3, orientaler Allotyp]
Ratte-IgG2b/Maus-[VH-CH1,VL-CL]-Human-IgG4-[Hinge-CH2-CH3]
Human-IgG1/Human-[VH-CH1,VL-CL]-Human-IgG1-[Hinge]-Human-IgG3*-[CH2-CH3]
Human-IgG1/Ratte-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]
Human-IgG1/Maus-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]
Human-IgG1/Ratte-[VH-CH1,VL-CL]-Human-IgG1-[Hinge]-Human-IgG2[N-terminale Region von CH2]-Human-IgG3*[C-terminale Region von CH2 : > AS-Position 251]-Human-IgG3*[CH3]
Human-IgG1/Maus-[VH-CH1,VL-CL]-Human-IgG1-[Hinge]-Human-IgG2[N-terminale Region von CH2]-Human-IgG3*[C-terminale Region von CH2 : > AS-Position 251]-Human-IgG3*[CH3]
Human-IgG1/Ratte-[VH-CH1,VL-CL]-Human-IgG1-[Hinge]-Human-IgG3*-[CH2-CH3]
Human-IgG1/Maus-[VH-CH1,VL-CL]-Human-IgG1-[Hinge]-Human-IgG3*-[CH2-CH3]
Human-IgG2/Human-[VH-CH1,VL-CL]-Human-IgG2-[Hinge]-Human-IgG3*-[CH2-CH3]
Human-IgG4/Human-[VH-CH1,VL-CL]-Human-IgG4-[Hinge]-Human-IgG3*-[CH2-CH3]
Human-IgG4/Human-[VH-CH1,VL-CL]-Human-IgG4-[Hinge]-Human-IgG4[N-terminale Region von CH2]-Human-IgG3*[C-terminale Region von CH2 : > AS-Position 251]-Human-IgG3*[CH3]
Maus-IgG2b/Ratte-[VH-CH1,VL-CL]-Human-IgG1-[Hinge]-Human-IgG3*-[CH2-CH3]
Maus-IgG2b/Human-[VH-CH1,VL-CL]-Human-IgG1-[Hinge]-Human-IgG3*-[CH2-CH3]
Maus-IgG2b/Maus-[VH-CH1,VL-CL]-Human-IgG1-[Hinge]-Human-IgG3*-[CH2-CH3]
Maus-[VH-CH1,VL-CL]-Human-IgG4/Ratte-[VH-CH1,VL-CL]-Human-IgG4-[Hinge]-Human-IgG4-[CH2]-Human-IgG3*-[CH3]
HumanIgG1/Ratte[VH-CH1,VL-CL]-Human-IgG1[Hinge]-Human-IgG4-[CH2]-HumanIgG3*[CH3]
HumanIgGl/Maus[VH-CH1,VL-CL]-Human-IgG1[Hinge]-Human-IgG4-[CH2]-Human-IgG3*[CH3]
Human-IgG4/Human[VH-CH1,VL-CL]-Human-IgG4-[Hinge]-Human-IgG4-[CH2]-HumanIgG3*-[CH3]

10. Verfahren nach einem oder mehreren der vorhergehenden Ansprüche,
dadurch gekennzeichnet, daß
der bispezifische Antikörper aus einem heterologen Ratte/Maus bispezifischen Antikörper ausgewählt wird.

11. Verfahren nach einem oder mehreren der vorhergehenden Ansprüche,
dadurch gekennzeichnet, daß
der trispezifische Antikörper einen T-Zell-Bindungsarm, einen Tumorzell-Bindungsarm und eine dritte Spezifität zur Bindung an Fc-Rezeptor positive Zellen besitzt.

12. Verfahren nach Anspruch 11,
dadurch gekennzeichnet, daß
der trispezifische Antikörper so ausgewählt wird, daß er ein anti-CD3 X anti-Tumor-assoziiertes Antigen- und/oder anti-CD4 X anti-Tumor-assoziiertes Antigen- und/oder anti-CD5 X anti-Tumor-assoziiertes Antigen- und/oder anti-CD6 X anti-Tumor-assoziiertes Antigen- und/oder anti-CD8 X anti-Tumor-assoziiertes Antigen- und/oder anti-CD2 X anti-Tumor-assoziiertes Antigen- und/oder anti-CD28 X anti-Tumor-assoziiertes Antigen- und/oder anti-CD44 X anti-Tumor-assoziiertes Antigen-Antikörper ist.

13. Verfahren nach einem oder mehreren der vorhergehenden Ansprüche,
dadurch gekennzeichnet, daß
im Schritt c) nach Inkubation der Tumorzellen mit intakten heterologen bispezifischen und/oder trispezifischen Antikörpern die mit den Antikörpern beladenen Tumorzellen zur Reinfusion vorbereitet werden (Kurzzeitinkubation).

14. Verfahren nach einem oder mehreren der Ansprüche 1 bis 12,
dadurch gekennzeichnet, daß
im Schritt c) die Inkubation der Tumorzellen mit den Antikörpern zusammen mit mononukleären Zellen aus dem peripheren Blut (PBMC = peripheral blood mononucleated cells) erfolgt oder nach Inkubation der Tumorzellen mit den Antikörpern die mononukleären Zellen zugegeben werden und die Inkubation fortgesetzt wird (Langzeitinkubation).

15. Verfahren nach Anspruch 13 oder 14,
dadurch gekennzeichnet, daß
die Tumorzellen mit den Antikörpern für einen Zeitraum von 10 Minuten bis 5 Stunden inkubiert werden.

16. Verfahren nach Anspruch 15,
dadurch gekennzeichnet, daß
die Inkubation der Tumorzellen mit den Antikörpern für einen Zeitraum von 15 bis 120 Minuten, bevorzugt 15 bis 60 Minuten, erfolgt.

17. Verfahren nach einem oder mehreren der vorhergehenden Ansprüche 14 bis 16,
dadurch gekennzeichnet, daß die mononukleären Zellen aus dem peripheren Blut zusammen mit den Tumorzellen und den Antikörpern für einen Zeitraum von 1 bis 14 Tagen, bevorzugt 3 bis 10 Tagen und weiterhin bevorzugt 6 bis 10 Tagen inkubiert werden.

18. Verfahren nach einem oder mehreren der vorhergehenden Ansprüche,
dadurch gekennzeichnet, daß
die mononukleären Zellen aus dem peripheren Blut in einer Menge von ca. 10⁸ bis 10¹⁰ Zellen zugegeben werden.

19. Verfahren nach einem oder mehreren der vorhergehenden Ansprüche,
dadurch gekennzeichnet, daß
die Tumorzellen in einer Menge von 10⁷ bis 10⁹ Zellen, bevorzugt ca. 10⁸ Zellen, zugegeben werden.

20. Verfahren nach einem oder mehreren der vorhergehenden Ansprüche,
dadurch gekennzeichnet, daß
die bispezifischen und/oder trispezifischen Antikörper in einer Menge von 2 bis 100 µg, bevorzugter 5 bis 70 µg, insbesondere bevorzugt 5 bis 50 µg zugegeben werden.

21. Verfahren nach einem oder mehreren der vorhergehenden Ansprüche,
dadurch gekennzeichnet, daß
die Behandlung der Tumorzellen im Schritt b durch Bestrahlung, bevorzugt durch γ-Bestrahlung, weiterhin bevorzugt in einer Dosisstärke von 50 bis 100 Gy, oder durch chemi sche Substanzen, bevorzugt Mitomycin C, erfolgt.

22. Verfahren nach einem oder mehreren der vorhergehenden Ansprüche, dadurch gekennzeichnet, daß die intakten heterologen bispezifischen und/oder trispezifischen Antikörper so ausgewählt werden, daß sie zur Aktivierung Fc-Rezeptorpositiver Zelle befähigt sind, wodurch die Expression von Cytokinen und/oder co-stimulatorischen Antigenen initiiert oder erhöht wird.

23. Verwendung der nach einem Verfahren nach einem oder mehreren der vorhergehenden Ansprüche hergestellten Tumorzellpräparate zur Herstellung eines Arzneimittels zur Prophylaxe und Behandlung von Tumorerkrankungen.

24. Verwendung nach Anspruch 23 zur Herstellung eines Arzneimittels zur Induktion einer Tumorimmunität.

25. Verwendung der nach einem Verfahren nach einem oder mehreren der vorhergehenden Ansprüche hergestellten Tumorzellpräparate zur Herstellung eines Arzneimittels zur Reinfusion in den Patienten oder das Tier, aus dem die autologen Tumorzellen entnommen wurden.

26. Pharmazeutische Zubereitung, enthaltend das nach einem Verfahren nach einem oder mehreren der vorhergehenden Ansprüche erhältliche Tumorzellpräparat, wahlweise zusammen mit weiteren, an sich üblichen Hilfs- und/oder Trägerstoffen.

## Claims

1. A method for the preparation of a tumour cell preparation for the immunization of humans and animals comprising the following steps of:
a) isolating autologous tumour cells;
b) treating the tumour cells to prevent the survival thereof following reinfusion;
c) incubating the thus treated tumour cells with intact heterologous bispecific and/or trispecific antibodies showing the following properties:
α - binding to a T cell;
β - binding to at least one antigen on a tumour cell;
γ - binding, by their Fc portion (in the case of bispecific antibodies), or by a third specificity (in the case of trispecific antibodies) to Fc receptor-positive cells.

2. A method according to claim 1,
characterized in that
said antibodies are selected so that they are capable of binding Fc receptor-positive cells having a Fcγ receptor I, II, or III.

3. A method according to claim 2,
characterized in that
said antibodies are capable of binding to monocytes, macrophages, dendritic cells, "natural killer" cells (NK cells) and/or activated neutrophils being Fcγ receptor I-positive cells.

4. A method according to one or more of the preceding claims,
characterized in that
said antibodies are capable of inducing tumour-reactive complement-binding antibodies and thus inducing a humoral immune response.

5. A method according to one or more of the preceding claims,
characterized in that
said antibodies are selected to bind to the T cells via CD2, CD3, CD4, CD5, CD6, CD8, CD28 and/or CD44.

6. A method according to one or more of the preceding claims,
characterized in that
said antibodies are selected so that following their binding to the Fc receptor-positive cells the expression of CD40, CD80, CD86, ICAM-1 and/or LFA-3 as co-stimulatory antigens, and/or secretion of cytokins by the Fc receptor-positive cell is initiated or increased.

7. A method according to claim 6,
characterized in that
said antibodies are selected so that the secretion of IL-1, IL-2, IL-4, IL-6, IL-8, IL-12 being cytokins and/or of TNF-α is increased.

8. A method according to one or more of the preceding claims,
characterized in that
said bispecific antibody is selected to be an anti-CD3 X anti-tumour-associated antigen antibody and/or anti-CD4 X anti-tumour-associated antigen antibody and/or anti-CD5 X anti-tumour-associated antigen antibody and/or anti-CD6 X anti-tumour-associated antigen antibody and/or anti-CD8 X anti-tumour-associated antigen antibody and/or anti-CD2 X anti-tumour-associated antigen antibody and/or anti-CD28 X anti-tumour-associated antigen antibody and/or anti-CD44 X anti-tumour-associated antigen antibody.

9. A method according to one or more of the preceding claims,
characterized in that
said bispecific antibody is selected from one or more of the following isotype combinations:
rat-IgG2b/mouse-IgG2a,
rat-IgG2b/mouse-IgG2b,
rat-IgG2b/mouse-IgG3;
rat-IgG2b/human-IgG 1,
rat-IgG2b/human-IgG2,
rat-IgG2b/human-IgG3 [oriental allotype G3m(st) = binding to protein A],
rat-IgG2b/human-IgG4;
rat-IgG2b/rat-IgG2c;
mouse-IgG2a/human-IgG3[caucasian allotypes G3m(b+g) = no binding to protein A, in the following indicated as *]
mouse-IgG2a/mouse-[VH-CH1,VL-CL]-human-IgG1-[hinge]-human-IgG3*-[CH2-CH3]
mouse-IgG2a/rat-[VH-CH1,VL-CL]-human-IgG1-[hinge]-human-IgG3*-[CH2-CH3]
mouse-IgG2a/human-[VH-CH1,VL-CL]-human-IgG1-[hinge]-human-IgG3*-[CH2-CH3]
mouse-[VH-CH1,VL-CL]-human-IgG1/rat-[VH-CH1,VL-CL]-human-IgG1-[hinge]-human-IgG3*-[CH2-CH3]
mouse-[VH-CH1,VL-CL]-human-IgG4/rat-[VH-CH1,VL-CL]-human-IgG4-[hinge]-human-IgG4[N-terminal region of CH2]-human-IgG3*[C-terminal region of CH2: > aa position 251]-human-IgG3*[CH3]
rat-IgG2b/mouse-[VH-CH1 ,VL-CL] -human-IgG1-[hinge-CH2-CH3]
rat-IgG2b/mouse-[VH-CH1,VL-CL]-human-IgG2-[hinge-CH2-CH3]
rat-IgG2b/mouse-[VH-CH1,VL-CL]-human-IgG3-[hinge-CH2-CH3, oriental allotype]
rat-IgG2b/mouse-[VH-CH1,VL-CL]-human-IgG4-[hinge-CH2-CH3]
human-IgG1/human-[VH-CH1,VL-CL]-human-IgG1-[hinge]-human-IgG3*-[CH2-CH3]
human-IgG1 /rat-[VH-CH1,VL-CL]-human-IgG1-[hinge]-human-IgG4[N-terminal region of CH2]-human-IgG3*[C-terminal region of CH2 : > aa position 251]-human-IgG3*[CH3]
human-IgG1 /mouse-[VH-CH 1,VL-CL]-human-IgG1-[hinge]human-IgG4[N-terminal region of CH2]-human-IgG3*[C-terminal region of CH2 : > aa position 251]-human-IgG3*[CH3]
human-IgG1/rat-[VH-CH1,VL-CL]-human-IgG1-[hinge]-human-IgG2[N-terminal region of CH2]-human-IgG3*[C-terminal region of CH2 : > aa position 251]-human-IgG3*[CH3]
human-IgG1/mouse-[VH-CH1,VL-CL]-human-IgG1-[hinge]-human-IgG2[N-terminal region of CH2]-human-IgG3*[C-terminal region of CH2 : > aa position 251]-human-IgG3*[CH3]
human-IgG1/rat-[VH-CH1,VL-CL]-human-IgG1-[hinge]-human-IgG3*-[CH2-CH3]
human-IgG1/mouse-[VH-CH1,VL-CL]-human-IgG1-[hinge]-human-IgG3*-[CH2-CH3]
human-IgG2/human-[VH-CH1,VL-CL]-human-IgG2-[hinge]-human-IgG3*-[CH2-CH3]
human-IgG4/human-[VH-CH1,VL-CL]-human-IgG4-[hinge]-human-IgG3 *-[CH2-CH3]
human-IgG4/human-[VH-CH1,VL-CL]-human-IgG4-[hinge]-human-IgG4[N-terminal region of CH2]-human-IgG3*[C-terminal region of CH2 : > aa position 251]-human-IgG3*[CH3]
mouse-IgG2b/rat-[VH-CH1,VL-CL]-human-IgG1-[hinge]-human-IgG3*-[CH2-CH3]
mouse-IgG2b/human-[VH-CH1,VL-CL]-human-IgG1-[hinge]-human-IgG3*-[CH2-CH3]
mouse-IgG2b/mouse-[VH-CH1,VL-CL]-human-IgG1-[hinge]-human-IgG3*-[CH2-CH3]
mouse-[VH-CH1,VL-CL]-human-IgG4/rat-[VH-CH1,VL-CL]-human-IgG4-[hinge]-human-IgG4-[CH2]-human-IgG3*-[CH3]
human-IgG1/rat-[VH-CH1,VL-CL]-human-IgG1-[hinge]-human-IgG4-[CH2]-human-IgG3*-[CH3]
human-IgG1/mouse-[VH-CH1,VL-CL]-human-IgG 1-[hinge]-human-IgG4-[CH2]-human-IgG3*-[CH3]
human-IgG4/human-[VH-CH1,VL-CL]-human-IgG4-[hinge]-human-IgG4-[CH2]-human-IgG3*-[CH3]

10. A method according to one or more of the preceding claims,
characterized in that
said bispecific antibody is selected from a heterologous rat/mouse bispecific antibody.

11. A method according to one or more of the preceding claims,
characterized in that
said trispecific antibody has a T cell binding arm, a tumour cell binding arm and a third specificity for binding to Fc receptor-positive cells.

12. A method according to claim 11,
characterized in that
said trispecific antibody is selected to be an anti-CD3 X anti-tumour-associated antigen antibody and/or anti-CD4 X anti-tumour-associated antigen antibody and/or anti-CD5 X anti-tumour-associated antigen antibody and/or anti-CD6 X anti-tumour-associated antigen antibody and/or anti-CD8 X anti-tumour-associated antigen antibody and/or anti-CD2 X anti-tumour-associated antigen antibody and/or anti-CD28 X anti-tumour-associated antigen antibody and/or anti-CD44 X anti-tumour-associated antigen antibody.

13. A method according to one or more of the preceding claims,
characterized in that
in said step c) after incubating the tumour cells with intact heterologous bispecific and/or trispecific antibodies the tumour cells charged with antibodies are prepared for reinfusion (short-term incubation).

14. A method according to one or more of the claims 1 to 12,
characterized in that
in said step c) the incubation of the tumour cells with antibodies is performed together with mononucleated cells of the peripheral blood (PBMC = peripheral blood mononucleated cells), or mononucleated cells are added after incubation of the tumour cells with the antibodies and the incubation is continued (long-term incubation).

15. A method according to claim 13 or 14,
characterized in that
said tumour cells are incubated with the antibodies for a period of 10 minutes to 5 hours.

16. A method according to claim 15,
characterized in that
said incubation of the tumour cells with the antibodies is performed for a period of 15 minutes to 120 minutes, preferably 15 minutes to 60 minutes.

17. A method according to one or more of the claims 14 to 16,
characterized in that
said mononucleated peripheral blood cells are incubated with the tumour cells and the antibodies for a period of 1 to 14 days, preferably 3 to 10 days, further preferred 6 to 10 days.

18. A method according to one or more of the preceding claims,
characterized in that
said mononucleated peripheral blood cells are added in an amount of about 10⁸ to 10¹⁰ cells.

19. A method according to one or more of the preceding claims,
characterized in that
said tumour cells are added in an amount of 10⁷ to 10⁹ cells, preferably about 10⁸ cells.

20. A method according to one or more of the preceding claims,
characterized in that
said bispecific and/or trispecific antibodies are added in an amount of 2 to 100 µg, preferably 5 to 70 µg, in particular preferred 5 to 50 µg.

21. A method according to one or more of the preceding claims,
characterized in that
said treating of the tumour cells in step b is performed by irradiation, preferably γ-irradiation, further preferred in a radiation dose of 50 to 100 Gy or by chemical substances, preferably mitomycin C.

22. A method according to one or more of the preceding claims,
characterized in that
said intact heterologous bispecific and/or trispecific antibodies are selected so that they are capable of activating the Fc receptor-positive cell whereby the expression of cytokins and/or co-stimulatory antigens is induced or increased.

23. Use of the tumour cell containing preparation prepared according to one or more of the preceding claims for the preparation of a medicament for the prevention and treatment of tumourous diseases.

24. Use according to claim 23 for the preparation of a medicament for inducing an antitumour immunity.

25. Use of the tumour cell preparations prepared according to one or more of the preceding claims for the preparation of a medicament for reinfusion into the patient or the animals from whom the autologous tumour cells have been obtained.

26. A pharmaceutical preparation containing a tumour cell preparation obtainable according to a method according to one or more of the preceding claims, optionally with further adjuvants and/or carriers which are common per se.

## Revendications

1. Procédé de fabrication d'une préparation de cellules tumorales pour l'immunisation d'humains et d'animaux, comprenant les étapes consistant à :
a) isoler des cellules tumorales autologues ;
b) traiter les cellules tumorales afin d'empêcher leur survie après reperfusion ;
c) incuber les cellules tumorales ainsi traitées avec des anticorps bispécifiques et/ou trispécifiques hétérologues intacts qui présentent les propriétés suivantes :
α - liaison à une cellule T ;
β - liaison à au moins un antigène sur une cellule tumorale ;
γ - liaison par l'intermédiaire de leur fragment Fc (dans le cas des anticorps bispécifiques) ou par l'intermédiaire d'une troisième spécificité (dans le cas des anticorps trispécifiques) à des cellules positives pour le récepteur du Fc.

2. Procédé selon la revendication 1, caractérisé en ce que les anticorps sont choisis de telle sorte qu'ils sont aptes à se lier à des cellules positives pour le récepteur du Fc, qui présentent un récepteur du Fcγ I, II ou III.

3. Procédé selon la revendication 2, caractérisé en ce que les anticorps sont aptes à se lier à des monocytes, des macrophages, des cellules dendritiques, des cellules tueuses naturelles (cellules NK) et/ou des cellules neutrophiles activées en tant que cellules positives pour le récepteur du Fcγ I.

4. Procédé selon l'une ou plusieurs des revendications précédentes, caractérisé en ce que les anticorps sont aptes à induire des anticorps liant le complément réactifs vis-à-vis de tumeurs et induire ainsi une réponse immunitaire humorale.

5. Procédé selon l'une ou plusieurs des revendications précédentes, caractérisé en ce que les anticorps sont choisis de telle sorte qu'ils se lient aux cellules T par l'intermédiaire du CD2, CD3, CD4, CD5, CD6, CD8, CD28 et/ou CD44.

6. Procédé selon l'une ou plusieurs des revendications précédentes, caractérisé en ce que les anticorps sont choisis de telle sorte que, après leur liaison aux cellules positives pour le récepteur du Fc, l'expression de CD40, CD80, CD86, ICAM-1 et/ou LFA-3 en tant qu'antigènes costimulants et/ou la sécrétion de cytokines par la cellule positive pour le récepteur du Fc est déclenchée ou augmentée.

7. Procédé selon la revendication 6, caractérisé en ce que les anticorps sont choisis de telle sorte que la sécrétion d'IL-1, IL-2, IL-4, IL-6, IL-8, IL-12 en tant que cytokines et/ou de TNF-α est augmentée.

8. Procédé selon l'une ou plusieurs des revendications précédentes, caractérisé en ce que l'anticorps bispécifique est choisi de telle sorte qu'il soit un anticorps anti-CD3 X anti-AAT (antigène associé aux tumeurs) et/ou un anticorps anti-CD4 X anti-AAT et/ou un anticorps anti-CD5 X anti-AAT et/ou un anticorps anti-CD6 X anti-AAT et/ou un anticorps anti-CD8 X anti-AAT et/ou un anticorps anti-CD2 X anti-AAT et/ou un anticorps anti-CD28 X anti-AAT et/ou un anticorps anti-CD44 X anti-AAT.

9. Procédé selon l'une ou plusieurs des revendications précédentes, caractérisé en ce que l'anticorps bispécifique est choisi parmi l'une ou plusieurs des combinaisons d'isotypes suivantes :
IgG2b de rat/IgG2a de souris,
IgG2b de rat/IgG2b de souris,
IgG2b de rat/IgG3 de souris,
IgG2b de rat/IgG1 humaine,
IgG2b de rat/IgG2 humaine,
IgG2b de rat/IgG3 humaine [allotype oriental G3m(st) = liaison à la protéine A],
IgG2b de rat/IgG4 humaine,
IgG2b de rat/IgG2c de rat,
IgG2a de souris/IgG3 humaine [allotypes caucasiens G3m(b+g) = pas de liaison à la protéine A, ci-après repérés par *],
IgG2a de souris/[VH-CH1,VL-CL] de souris-IgG1 humaine-[segment de liaison]-IgG3* humaine-[CH2-CH3],
IgG2a de souris/[VH-CH1,VL-CL] de rat-IgG1 humaine[segment de liaison]-IgG3* humaine-[CH2-CH3],
IgG2a de souris/[VH-CH1,VL-CL] humaine-IgGl humaine[segment de liaison]-IgG3* humaine-[CH2-CH3],
[VH-CH1,VL-CL] de souris-IgGl humaine/[VH-CH1,VL-CL] de rat-IgGl humaine-[segment de liaison]-IgG3* humaine-[CH2-CH3],
[VH-CH1,VL-CL] de souris-IgG4 humaine/[VH-CH1,VL-CL] de rat-IgG4 humaine-[segment de liaison]-IgG4 humaine [région N-terminale de CH2]-IgG3* humaine [région C-terminale de CH2 : > position d'acide aminé 251]-IgG3* humaine-[CH3],
IgG2b de rat/[VH-CH1,VL-CL] de souris-IgGl humaine [segment de liaison-CH2-CH3],
IgG2b de rat/[VH-CH1,VL-CL] de souris-IgG2 humaine [segment de liaison-CH2-CH3],
IgG2b de rat/[VH-CH1,VL-CL] de souris-IgG3 humaine[segment de liaison-CH2-CH3, allotype oriental],
IgG2b de rat/[VH-CH1,VL-CL] de souris-IgG4 humaine[segment de liaison-CH2-CH3],
IgG1 humaine/[VH-CH1,VL-CL] humaine-IgG1 humaine[segment de liaison]-IgG3* humaine-[CH2-CH3],
IgG1 humaine/[VH-CH1,VL-CL] de rat-IgG1 humaine[segment de liaison]-IgG4 humaine [région N-terminale de CH2]-IgG3* humaine [région C-terminale de CH2 : > position d'acide aminé 251]-IgG3* humaine-[CH3],
IgG1 humaine/[VH-CH1,VL-CL] de souris-IgG1 humaine[segment de liaison]-IgG4 humaine [région N-terminale de CH2]-IgG3* humaine [région C-terminale de CH2 : > position d'acide aminé 251]-IgG3* humaine-[CH3],
IgG1 humaine/[VH-CH1,VL-CL] de rat-IgG1 humaine[segment de liaison]-IgG2 humaine [région N-terminale de CH2]-IgG3* humaine [région C-terminale de CH2 : > position d'acide aminé 251]-IgG3* humaine-[CH3],
IgG1 humaine/[VH-CH1,VL-CL] de souris-IgG1 humaine[segment de liaison]-IgG2 humaine [région N-terminale de CH2]-IgG3* humaine [région C-terminale de CH2 : > position d'acide aminé 251]-IgG3* humaine-[CH3],
IgG1 humaine/[VH-CH1,VL-CL] de rat-IgG1 humaine[segment de liaison]-IgG3* humaine-[CH2-CH3],
IgG1 humaine/[VH-CH1,VL-CL] de souris-IgG1 humaine[segment de liaison]-IgG3* humaine-[CH2-CH3],
IgG2 humaine/[VH-CH1,VL-CL] humaine-IgG2 humaine[segment de liaison]-IgG3* humaine-[CH2-CH3],
IgG4 humaine/[VH-CH1,VL-CL] humaine-IgG4 humaine[segment de liaison]-IgG3* humaine-[CH2-CH3],
IgG4 humaine/[VH-CH1,VL-CL] humaine-IgG4 humaine[segment de liaison]-IgG4 humaine [région N-terminale de CH2]-IgG3* humaine [région C-terminale de CH2 : > position d'acide aminé 251]-IgG3* humaine-[CH3],
IgG2b de souris/[VH-CH1,VL-CL] de rat-IgG1 humaine[segment de liaison]-IgG3* humaine-[CH2-CH3],
IgG2b de souris/[VH-CH1,VL-CL] humaine-IgG1 humaine[segment de liaison]-IgG3* humaine-[CH2-CH3],
IgG2b de souris/[VH-CH1,VL-CL] de souris-IgG1 humaine-[segment de liaison]-IgG3* humaine-[CH2-CH3],
[VH-CH1,VL-CL] de souris-IgG4 humaine/[VH-CH1,VL-CL] de rat-IgG4 humaine-[segment de liaison]-IgG4 humaine-[CH2]-IgG3* humaine-[CH3],
IgG1 humaine/[VH-CH1,VL-CL] de rat-IgG1 humaine[segment de liaison]-IgG4 humaine-[CH2]-IgG3* humaine-[CH3],
IgG1 humaine/[VH-CH1,VL-CL] de souris-IgG1 humaine[segment de liaison]-IgG4 humaine-[CH2]-IgG3* humaine-[CH3],
IgG4 humaine/[VH-CH1,VL-CL] humaine-IgG4 humaine[segment de liaison]-IgG4 humaine-[CH2]-IgG3* humaine-[CH3],

10. Procédé selon l'une ou plusieurs des revendications précédentes, caractérisé en ce que l'anticorps bispécifique est choisi parmi un anticorps bispécifique hétérologue de rat/souris.

11. Procédé selon l'une ou plusieurs des revendications précédentes, caractérisé en ce que l'anticorps trispécifique possède un bras de liaison aux cellules T, un bras de liaison aux cellules tumorales et une troisième spécificité de liaison à des cellules positives pour le récepteur du Fc.

12. Procédé selon la revendication 11, caractérisé en ce que l'anticorps trispécifique est choisi de telle sorte qu'il soit un anticorps anti-CD3 X anti-AAT (antigène associé aux tumeurs) et/ou un anticorps anti-CD4 X anti-AAT et/ou un anticorps anti-CD5 X anti-AAT et/ou un anticorps anti-CD6 X anti-AAT et/ou un anticorps anti-CD8 X anti-AAT et/ou un anticorps anti-CD2 X anti-AAT et/ou un anticorps anti-CD28 X anti-AAT et/ou un anticorps anti-CD44 X anti-AAT.

13. Procédé selon l'une ou plusieurs des revendications précédentes, caractérisé en ce que, à l'étape c), après incubation des cellules tumorales avec des anticorps bispécifiques et/ou trispécifiques hétérologues intacts, les cellules tumorales chargées avec les anticorps sont préparées pour la reperfusion (incubation de courte durée).

14. Procédé selon l'une ou plusieurs des revendications 1 à 12, caractérisé en ce que, à l'étape c), l'incubation des cellules tumorales avec les anticorps se fait ensemble avec des cellules PBMC (peripheral blood mononucleated cells = cellules mononucléaires du sang périphérique) ou bien les cellules mononucléaires sont ajoutées après l'incubation des cellules tumorales avec les anticorps et l'incubation est poursuivie (incubation de longue durée).

15. Procédé selon la revendication 13 ou la revendication 14, caractérisé en ce que les cellules tumorales sont incubées avec les anticorps pendant une durée de 10 minutes à 5 heures.

16. Procédé selon la revendication 15, caractérisé en ce que l'incubation des cellules tumorales avec les anticorps se fait pendant une durée de 15 à 120 minutes, de préférence 15 à 60 minutes.

17. Procédé selon l'une ou plusieurs des revendications 14 à 16 précédentes, caractérisé en ce que les cellules mononucléaires provenant du sang périphérique sont incubées ensemble avec les cellules tumorales et les anticorps pendant une durée de 1 à 14 jours, de préférence de 3 à 10 jours et de préférence encore de 6 à 10 jours.

18. Procédé selon l'une ou plusieurs des revendications précédentes, caractérisé en ce que les cellules mononucléaires provenant du sang périphérique sont ajoutées en une quantité de 10⁸ à 10¹⁰ cellules environ.

19. Procédé selon l'une ou plusieurs des revendications précédentes, caractérisé en ce que les cellules tumorales sont ajoutées en une quantité de 10⁷ à 10⁹ cellules, de préférence 10⁸ cellules environ.

20. Procédé selon l'une ou plusieurs des revendications précédentes, caractérisé en ce que les anticorps bispécifiques et/ou trispécifiques sont ajoutés en une quantité de 2 à 100 µg, de préférence de 5 à 70 µg, et de façon particulièrement préférée de 5 à 50 µg.

21. Procédé selon l'une ou plusieurs des revendications précédentes, caractérisé en ce que le traitement des cellules tumorales à l'étape b) se fait par exposition à un rayonnement, de préférence un rayonnement γ, de préférence encore à un niveau de dose de 50 à 100 Gy, ou bien à l'aide de substances chimiques, de préférence la mitomycine C.

22. Procédé selon l'une ou plusieurs des revendications précédentes, caractérisé en ce que les anticorps bispécifiques et/ou trispécifiques hétérologues intacts sont choisis de telle sorte qu'ils soient aptes à activer les cellules positives pour le récepteur du Fc, ce qui a pour effet de déclencher ou augmenter l'expression de cytokines et/ou d'antigènes costimulants.

23. Utilisation de la préparation de cellules tumorales fabriquée par un procédé selon l'une ou plusieurs des revendications précédentes pour la fabrication d'un médicament pour la prévention et le traitement de maladies tumorales.

24. Utilisation selon la revendication 23 pour la fabrication d'un médicament destiné à induire une immunité tumorale.

25. Utilisation de la préparation de cellules tumorales fabriquée par un procédé selon l'une ou plusieurs des revendications précédentes pour la fabrication d'un médicament destiné à être réinjecté au patient ou à l'animal chez lequel les cellules tumorales autologues ont été prélevées.

26. Préparation pharmaceutique contenant la préparation de cellules tumorales pouvant être obtenue par un procédé selon l'une ou plusieurs des revendications précédentes, le cas échéant ensemble avec d'autres additifs et/ou véhicules en soi usuels.
